# EUROPEAN PATENT APPLICATION

(11) **EP 4 660 317 A1**
(43) Date of publication of application: **10.12.2025**
(21) Application number: 25180326.8
(22) Date of filing: 03.06.2025
(51) Int. Cl.: C12Q 1/6806

(54) **METHOD FOR BARCODING MOLECULES IN SINGLE-CELL EXPERIMENTS**

(30) Priority: 05.06.2024 EP 24180195
(71) Applicant: Miltenyi Biotec B.V. & Co. KG, 51429 Bergisch Gladbach (DE)
(72) Inventor: Bernhard Wahl, Matthias, 51429 Bergisch Gladbach (DE)

(57) **Abstract**

The current invention provides a cost effective and reliable method for barcoding of target nucleic acids from single cells.

## Description

### Field of the invention

The invention belong to the field of library preparation and barcoding of nucleic acids. Key applications are among others single cell sequencing and/or antibody sequencing.

### Background of the invention

Methods for analyzing nucleic acids on single cell level are increasingly exploited in biological and biomedical research. Such methods allow understanding the heterogeneity of tissues or cell populations, and can be utilized to identify sub-populations of cells implicated in disease.

To facilitate single cell analysis and enable multiplexing, current state in the art workflows include two essential features: partitioning of the each single cell and barcoding of cell specific target nucleic acids.

Different types of barcodes, are known in the art such as cellular barcodes, sample specific barcodes and unique molecular identifier (UMI). For example cellular barcodes are used to assign target nucleic acids to their cell source. When using cellular barcodes, all target nucleic acids from one cell carry the same barcode sequence, wherein all target nucleic acids from another cell carry a different barcode. Based on that, target nucleic acids can be identified in a cell specific manner. In contrast to that, unique molecular identifier are used to identify single target nucleic acids. In an experiment each target nucleic acid may carry a different barcode. Moreover, dependent on the application, sample specific barcodes may be used, to identify which target nucleic acid originates from which sample. In standard sequencing experiments the different barcode types are combined to enable multiplexing. During the analysis step of the sequencing data, these barcodes can be utilized to distinguish different target molecules (based upon the unique molecular identifier), to assign individual reads to the cell it originates from, and/or to a particular sample.

Current methods for analyzing single biological particles, including single cells, use either droplets (water-in-oil emulsions) or small vessels for partitioning these particles and subsequently incorporating a specific barcode into the target nucleic acid molecules within these partitions. Based on that, barcoded target nucleic acid molecules from one cell can be distinguished from target nucleic acid molecules from another cell during a cell analysis step, such as next generation sequencing. One example is the analysis of different mRNA transcripts expressed in individual cells in a highly parallel fashion; the principle can also be applied to the analysis of genomic DNA or the presence of proteins or other biomolecules by using barcoded binders.

During sequence analysis, the barcode can be used to associate nucleic acids derived from the same cell.

Exemplary workflows for these methods are e.g. the single-cell portfolio of 10x Genomics (Chromium-based); InDrop single-cell Next Generation transcript sequencing (Klein et al, 2015), Drop-seq single-cell Next Generation transcript sequencing (Macosco et al, 2015).

One of the major drawbacks of current state in the art methods is that barcode molecules (or oligonucleotides comprising specific barcode sequences or combinations) have to be provided in high concentrations and for cellular barcodes in multiple copies to the target nucleic acids, otherwise not every target nucleic acid will be barcoded. This in turn means, that a lot of material (barcoding oligonucleotides) is needed, which is very cost intensive. Moreover most methods for barcoding are bead based. Bead based methods are not only expensive but also time consuming, as bead synthesis is complicated, error prone and time consuming.

For this reason, there is a need in the art for an improved or alternative, cheap and reliable method for barcoding target nucleic acids.

### Brief description of the invention

The current invention provides a cost effective and reliable method for barcoding of target nucleic acids from single cells.

Key element, of the method is the co-partitioning of single cells or biological particles with single copies of first and second oligonucleotides comprising different barcode sequences. The barcode sequences are different among the partitions. Importantly the first plurality of oligonucleotides may be provided in excess compared to the second plurality of oligonucleotides. After releasing the target nucleic acids of the biological particles, the first oligonucleotides are attached to the target nucleic acids. Based on that, each target nucleic acid carries a different oligonucleotide which comprises a different barcode sequence (similar to a UMI sequence). There follows an amplification step, in which the barcoded target nucleic acids and the second oligonucleotides are amplified, thereby generating copies of the second oligonucleotides and copies of the barcoded target nucleic acids. After amplification the copies of the second oligonucleotides are attached to the copies of the barcoded target nucleic acids. Finally the target nucleic acids carry a first and a second barcode sequence, which make up cell or partition specific combinations. After sequencing these combinations can be reassigned to the cell source or partition.

In a first aspect, the invention provides a method for barcoding of nucleic acids comprising the steps: A) Providing a plurality of biological particles comprising target nucleic acids, a first plurality of oligonucleotides comprising a first barcode sequence, wherein each oligonucleotide of the first plurality of oligonucleotides comprises a different barcode sequence and a second plurality of oligonucleotides comprising a second barcode sequence, wherein each oligonucleotide of the second plurality of oligonucleotides comprises a different barcode sequence. B) Partitioning the plurality of biological particles such that each partition comprises a biological particle and a subset of the first and second oligonucleotides. C) (Optionally) Releasing the target nucleic acids of the biological particles into the partition. D) Attaching to each target nucleic acid one first oligonucleotide, thereby generating barcoded target nucleic acids, wherein each target nucleic acid comprises a first barcode sequence, wherein this first barcode sequence is different for each target nucleic acid. E) Amplifying the second plurality of oligonucleotides and the barcoded target nucleic acids, thereby generating multiple copies of the second plurality of oligonucleotides and of the target nucleic acids. F) Attaching the second plurality of oligonucleotides to the target nucleic acids, thereby generating combinatorial barcoded target nucleic acids, wherein each target nucleic acid comprises a specific combination of the first and second specific barcode sequences, wherein the specific combination of the first and second barcodes serves as partition specific barcode.

### Brief description of the drawings

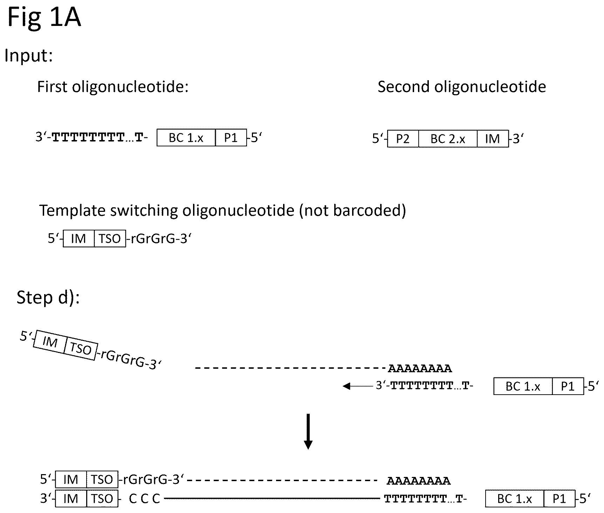

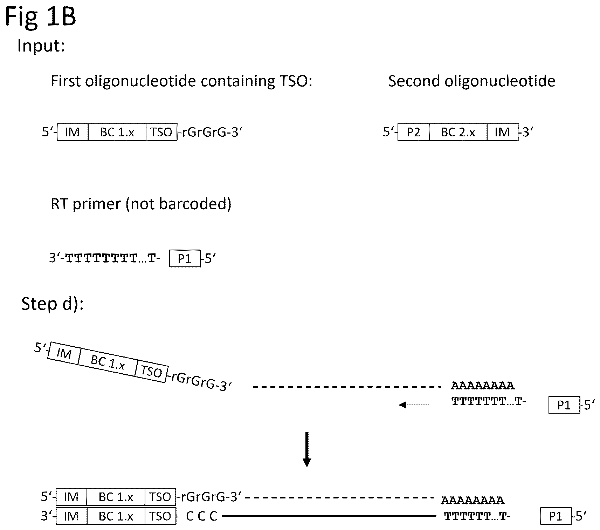

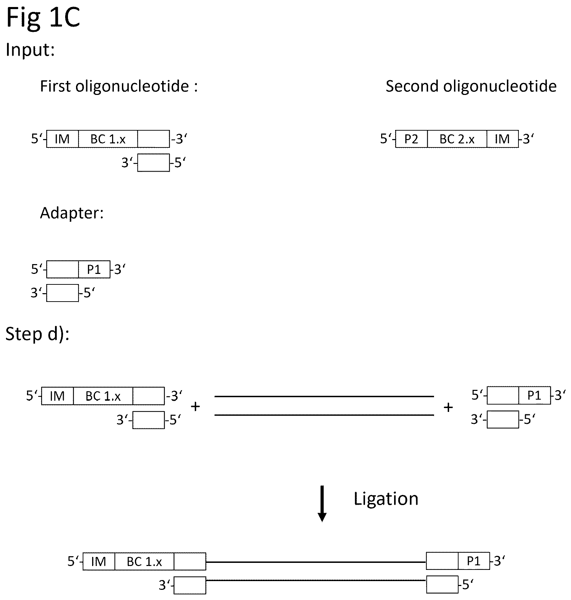

**Figure 1****: Possible structure of first and second oligonucleotide.** Legend Fig 1A-C: P1 (PS1) and P2 (PS2): specific primer binding sites; BC1.x: barcode of first oligonucleotide; BC2.x: barcode of second oligonucleotide; TSO: template switching oligo. Dashed lines depict RNA, solid lines or boxes depict DNA. In Fig 1A - B. The target nucleic acid is depicted as a dotted line and the barcoded cDNA (copy of the target nucleic acid) is depicted as solid line. In Fig. 1C The target nucleic acid is double stranded and depicted as solid lines
   Fig. 1A: barcoding of mRNA/ cDNA as target nucleic acid. The first oligonucleotide comprises the first barcode (BC1.x) and an oligo(dT) sequence. The first oligonucleotide is used as primer for cDNA synthesis, thereby generating a barcoded cDNA (copy of target nucleic acid) during step d). A primer binding sequence (PS1 or P1) is added to the barcoded nucleic acid (barcoded cDNA) by template switching using the template switching oligonucleotide. Both oligonucleotides (first and second) contain an intermediate sequence (IM) which can be used as primer binding site for amplification in step e) and joining the copies of barcoded target nucleic acid and second oligonucleotide with barcode in step f).
   Fig. 1B: barcoding of mRNA/ cDNA as target nucleic acid. mRNA is converted to cDNA using an oligonucleotide containing a oligo(dT) sequence and a primer binding site (P1 or PS1). The barcode is incorporated into the cDNA by template switching using a first oligonucleotide containing the first barcode (BC1.x), an intermediate sequence (IM) and a template switching oligo sequence (step D).
   Fig. 1C: barcoding of dsDNA by ligation. The first oligonucleotide comprises the first barcode (BC1.x), a intermediate sequence (IM) and a double-stranded region to facilitate ligation by T4 ligase (or a similar enzyme) (step D). A second (at least partially double-stranded) adapter is added to the reaction to generate a barcoded nucleic acid containing two primer binding sites (IM and PS1).

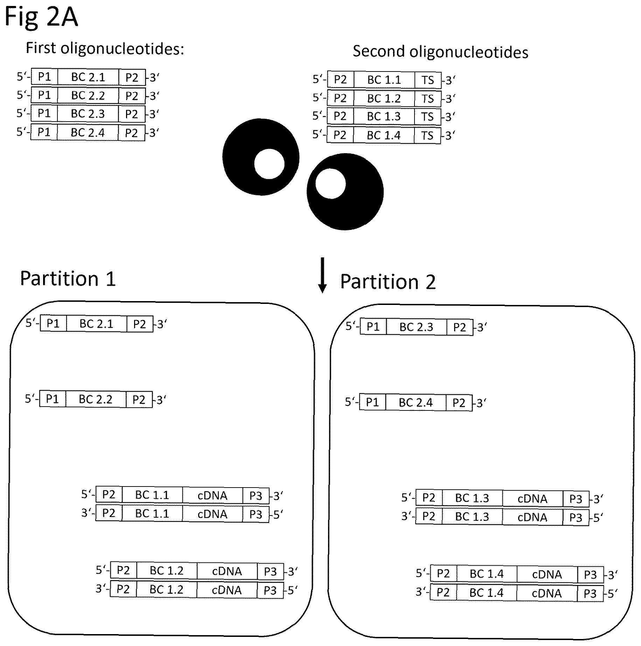

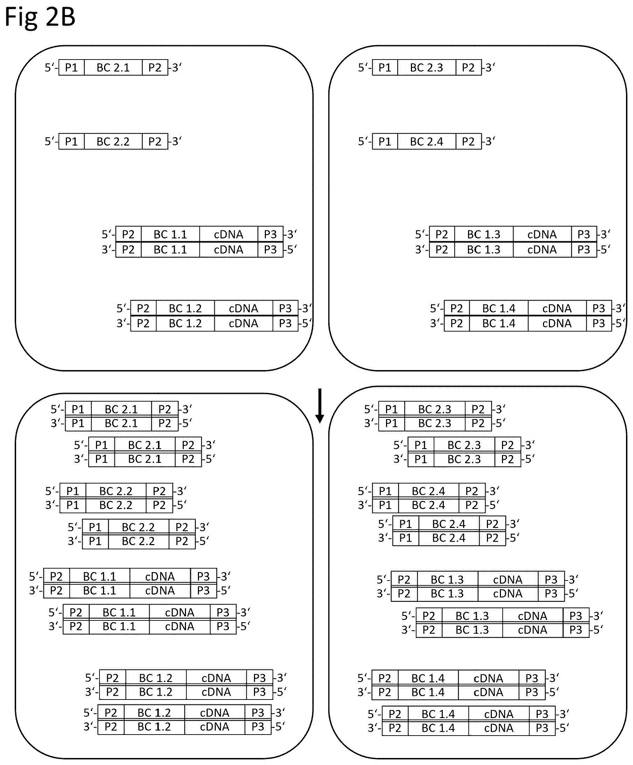

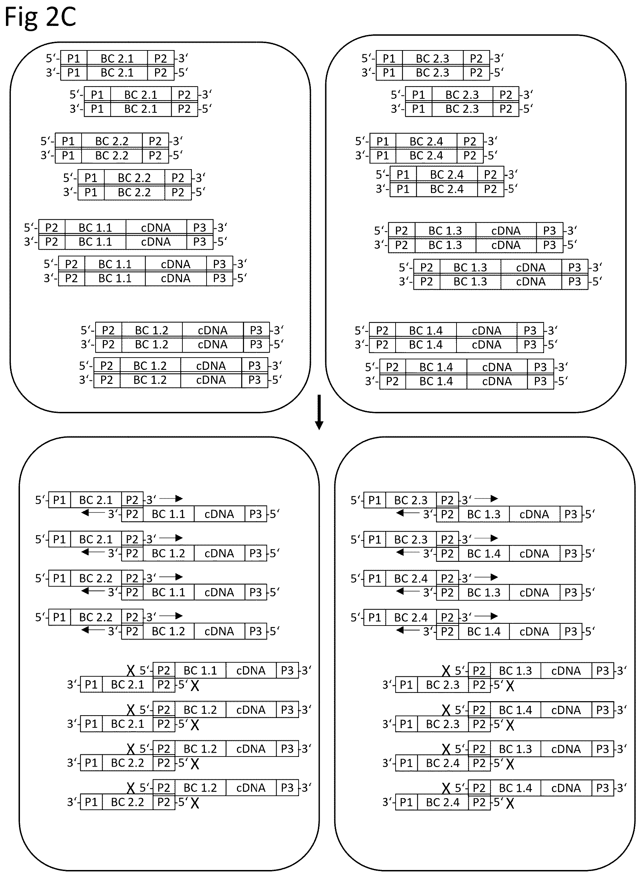

**Figure 2****: Principle of Method, example cDNA workflow.** Input: multiple copies of a first oligonucleotide (containing a first random barcode [depicted as BC1.x, where each number for x indicates a different sequence], a primer binding site P2 (PS2) and a sequence to facilitate template switching [TS]), and multiple copies of a second oligonucleotide (containing a second random barcode [depicted as BC2.x, where each number for x indicates a different sequence] flanked by two primer binding sites P1 (PS1) and P2 (PS2)) and cells. Fig 2A: Step 1: The input is partitioned (statistical partitioning or active partitioning), thereby generating partitions with a subset of the first oligonucleotide, the second oligonucleotide and the cells. Ideally, each partition contains a single cell. In the partition, the cells are lysed and the mRNA is converted into cDNA using the first oligonucleotide and a third oligonucleotide containing a primer for cDNA synthesis and a third primer binding site P3 (PS3). Fig 2B: Step 2 (Initial amplification of oligonucleotide containing first barcode and cDNA containing second barcode): The copies of the second oligonucleotide and the barcoded cDNA are amplified in the partition (using primer pairs for the primer binding sites P1/P2 (PS1/PS2) and P2/P3 (PS2/PS3). Fig 2C: Step 3 (Denaturation and random hybridization of amplicons at second primer binding site → extension to generate ds hybrid molecules): The amplicons generated in step 2 are randomly combined via the common primer binding site P2 (PS2). After initial extension, the hybrid molecules can optionally be amplified using primer pairs for the primer binding sites P1/P3 (PS2/PS3). As a result, partition-specific combinations of first and second barcode will be generated. By analyzing sufficient molecules, enough partition-specific combinations will be obtained in order to assign the cDNA to separate partitions.

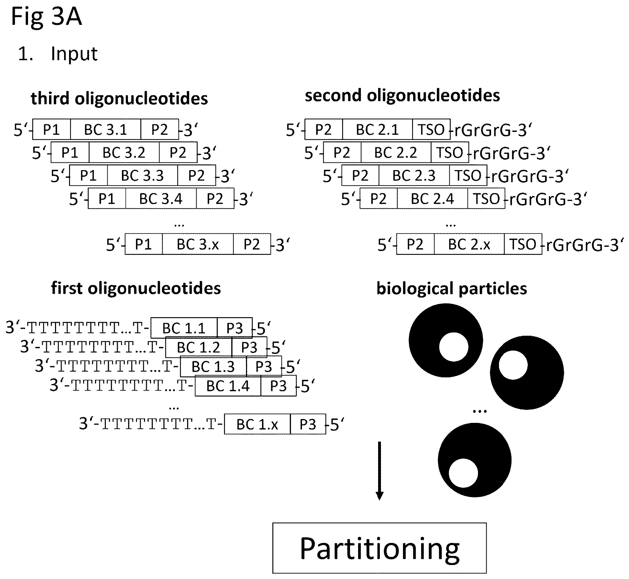

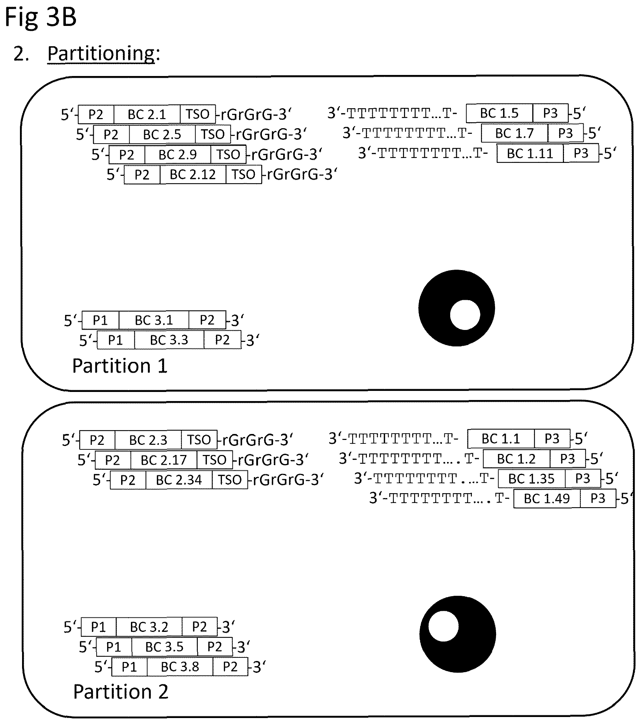

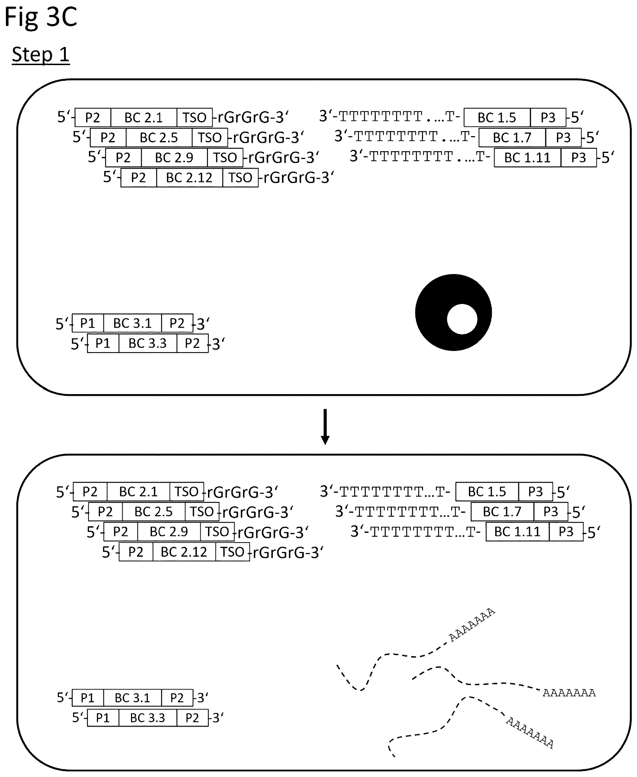

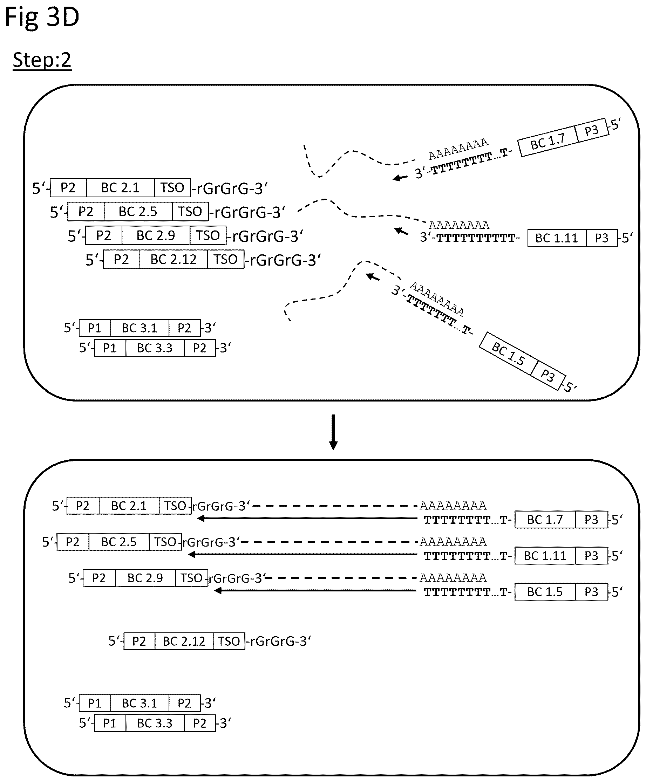

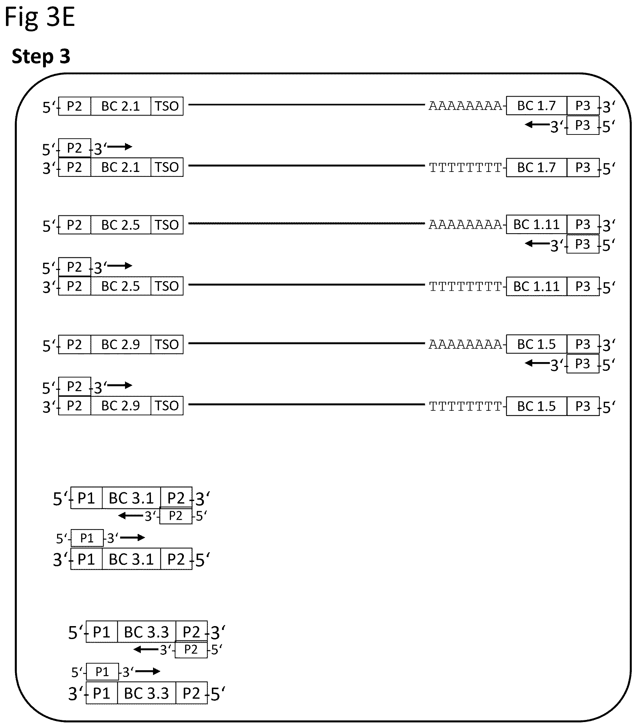

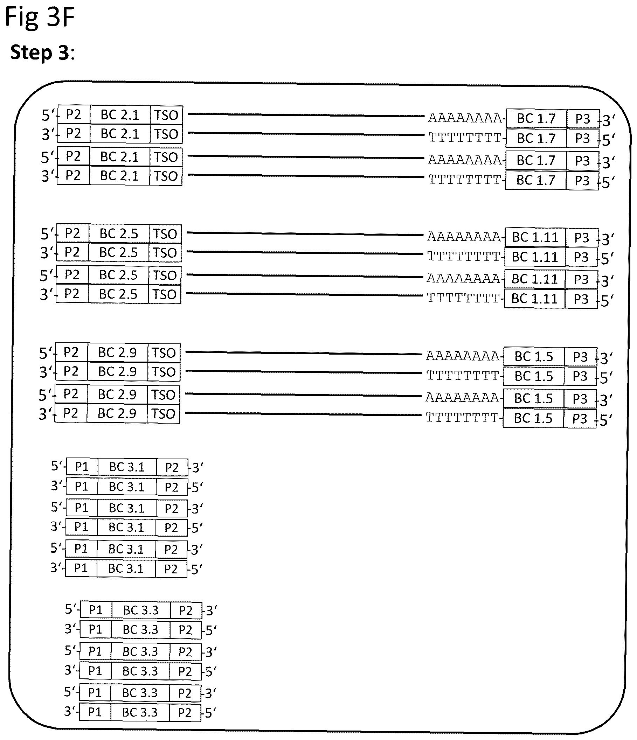

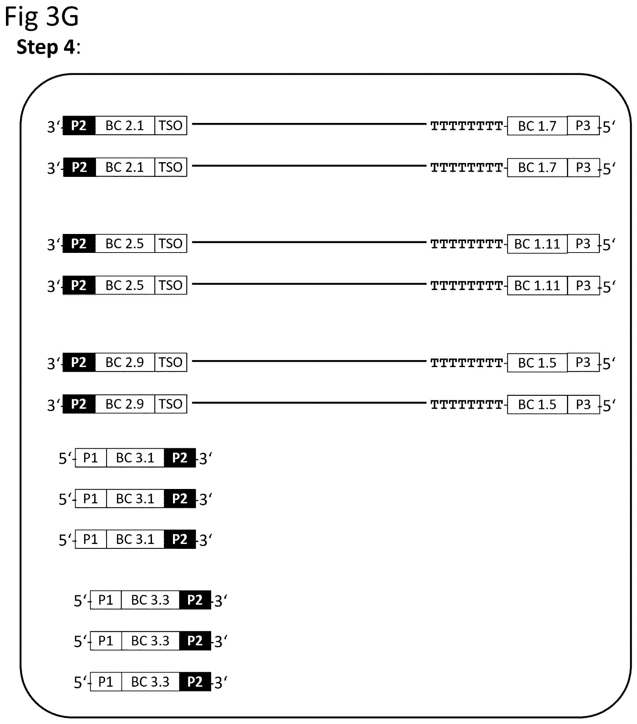

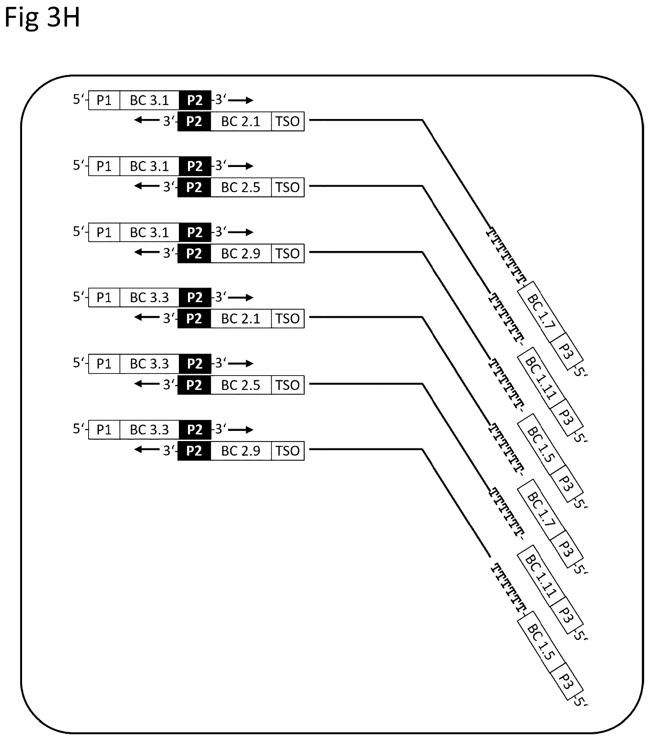

**Figure 3****: Example for workflow using three barcodes.**
   Fig. 3A: Input: multiple copies of a first oligonucleotide (containing a first random barcode [depicted as BC1.x, where each number for x indicates a different sequence], a capture sequence [the figure uses the example of an oligo(dT) primers], and a third primer binding site P3 (PS3); multiple copies of a second oligonucleotide (containing a second random barcode [depicted as BC2.x, where each number for x indicates a different sequence], a primer binding site P2 (PS2) and a sequence to facilitate template switching [TSO]) and multiple copies of a third oligonucleotide (containing a third random barcode [depicted as BC3.x, where each number for x indicates a different sequence] flanked by two primer binding sites P1 (PS1) and P2 (PS2)); multiple biological particles, e.g. cells. Note: In Fig 3B ff, an exemplary barcode using cells is shown. In the first step, the input is partitioned into multiple compartments (partitions).
   Fig. 3B: After partitioning, each partition contains a random subset of the multiple copies of the first oligonucleotide, multiple copies of the second oligonucleotide, multiple copies of the third oligonucleotide, and the biological particles (cells).
   Fig 3C - 3H: Reaction cascade within partition. Note: In Fig 3C ff, the process is continued using the example of the first partition of Figure 3B
   Fig 3C: Step 1: The cell is lysed and its mRNA is released into the partition.
   Fig 3D: Step 2: The mRNA is converted into cDNA by reverse transcription using the barcoded first oligonucleotide as primer and the barcoded second oligonucleotide as template switching oligonucleotide. The resulting cDNA will contain specific barcodes at the 5' and 3' end.
   Fig. 3E/3F: Step 3: The third oligonucleotide and the barcoded cDNA molecules generated in Step 2 are amplified separately. Fig. 3E depicts the nucleic acids after annealing of the amplification primers, and Fig 3F depicts the nucleic acids after the first amplification cycle.
   Fig. 3G/3H: Step 4 (denaturation followed by combination of different constructs via hybridization to second primer binding site): After Step 3, amplicons are made single-stranded (for example, by using heat). The resulting single-stranded copies of the third oligonucleotides and the copies of the barcoded cDNA molecules both contain the second primer binding site (full sequence or partial sequence), which can lead to a hybridization between the copies of the oligonucleotides and the copies of the barcoded cDNA molecules (Fig 3H). Those hybrids are extended using a DNA polymerase. Note: for simplicity, only one strand of each amplicon are depicted (the strands which can form extendable hybrid molecules).

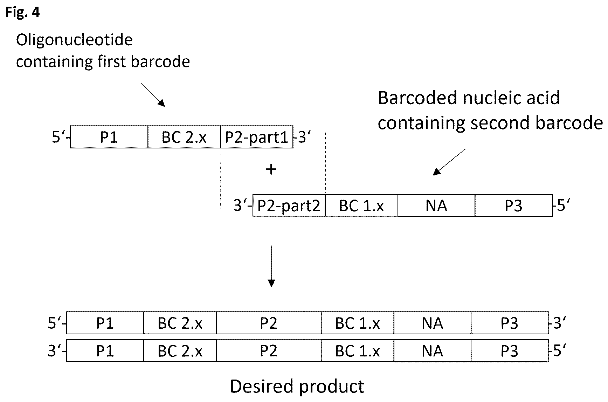

**Figure 4****: Method for separately amplifying barcode-containing oligonucleotides (or its derivatives) followed by combination by hybridization and extension (Method to amplify barcode containing fragments followed by random combination - structure of constructs).** The top of the figure shows the structure of the first oligonucleotide and the second nucleotide, and the bottom of the figure shows the structure of the resulting molecule after combination. The first and the second oligonucleotide contain only a partial sequence of the second primer site (depicted P2-part1 and P2-part2). Both P2-part1 and P2-part2 overlap, but the overlap is only partial.
   Initially, the barcode containing constructs are amplified separately using specific primer pairs (P1 + P2-part1, and P2-part2 + P3, separately). The annealing temperate is chosen so that P2-part1 and P2-part2 can hybridize to the two barcode containing constructs, but lower than the annealing temperature of P2-part1 and P2-part2 with each other (since they only partially overlap). After initial amplification, the annealing temperature is lowered to allow hybridizing of P2-part1 containing nucleic acids and P2-part2 containing nucleic acids. This facilitates hybridization of the separately amplified constructs, which can be extended to generated double-stranded hybrid constructs. The desired product is generated (random combination of oligonucleotide containing first barcode and barcoded nucleic acid containing second barcode)

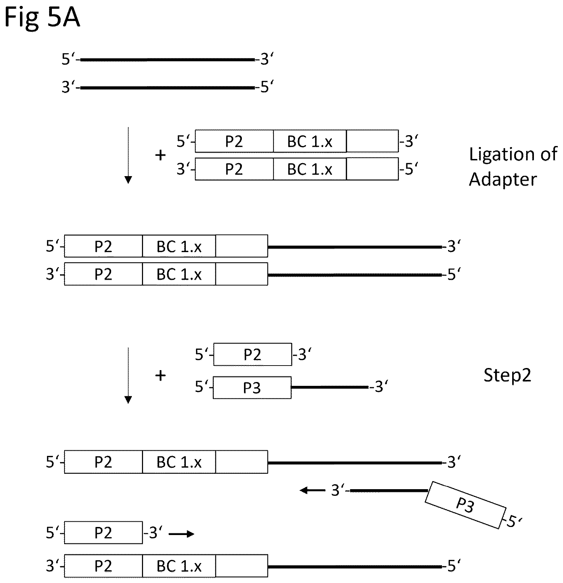

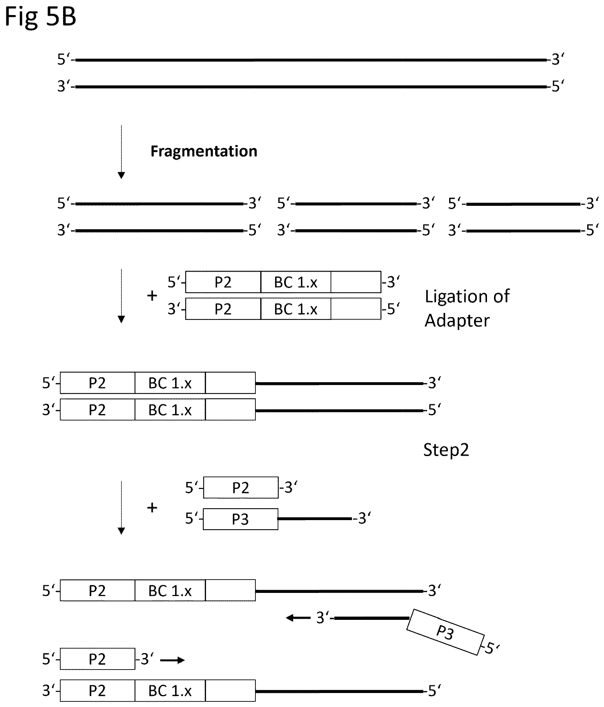

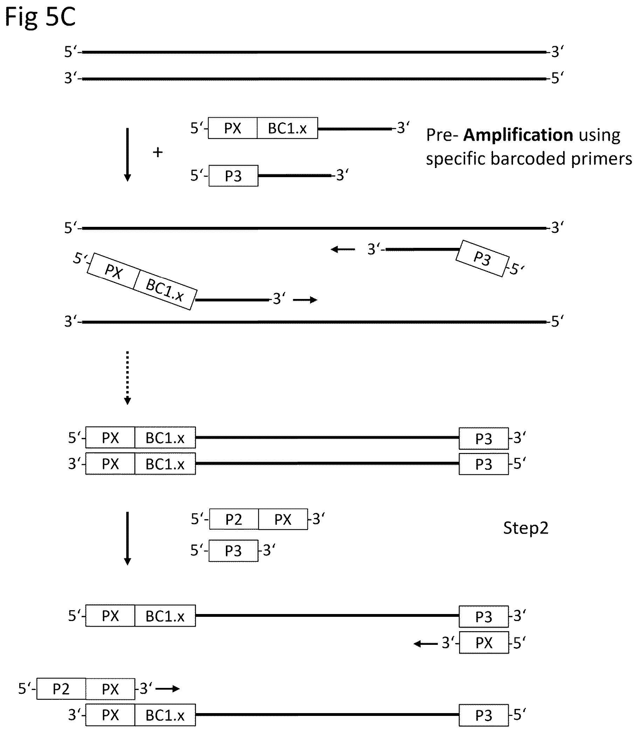

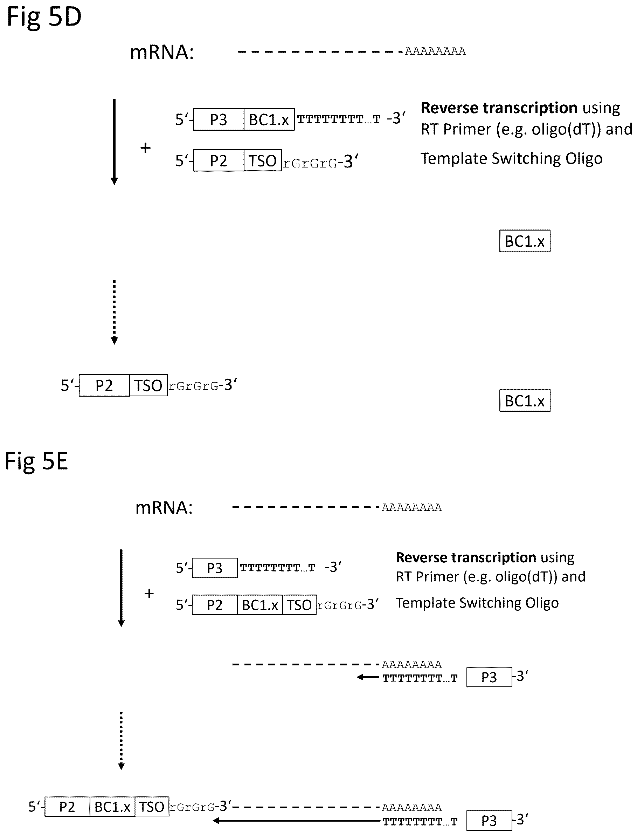

**Figure 5****: Method for barcoding nucleic acids.** Figures 5A to 5E show different methods for linking a barcode to a nucleic acid. In each figure, the barcode is depicted as BC1.x, where each number for x indicates a different sequence. The barcode may be directly linked to the nucleic acid by ligation (Fig 5A and B), or during the synthesis of a copy of the target nucleic acid.
   Fig 5A: Barcoding by ligation: The barcode may be added by ligating a barcode containing adapter to one (shown here) or both sides of a nucleic acid fragment. These barcoded fragments can be amplified during the initial amplification step (step 2 of proposed method) using suitable primers (universal primers specific for the adapter and/or primer specific for the barcoded nucleic acid).
   Fig 5B: Fragmentation followed by barcoding by ligation: Some samples may require fragmentation before ligating a barcoded adapter. For example, in approaches analyzing genomic DNA, the genomic DNA is often fragmented before ligating the adapter. The barcode may be added by ligating a barcode containing adapter to one (shown here) or both sides of a nucleic acid fragment. These barcoded fragments can be amplified during the initial amplification step (step 2 of proposed method) using suitable primers (universal primers specific for the adapter and/or primer specific for the barcoded nucleic acid).
   Fig 5C: Barcoding by primer extension (By limited pre-amplification): The adapter may also be added by a primer extension step. For example, primers may be used containing a primer binding site specific to the target nucleic acid (depicted with the black line), a barcode sequence (BC1.x), and a universal primer binding site (PX). This primer may be used for a primer extension reaction (single cycle or multiple cycles). The resulting barcoded nucleic acids subsequently serves as template in the initial amplification step (step 2) of the proposed method.
   Fig 5D and 5E: Barcoding by template switching: mRNA molecules may be indirectly barcoded during cDNA synthesis. In one approach, the barcode may be introduced by template switching (Zhu et al., 2001) using a barcode containing template switching oligo (Fig 5D). In another approach, the barcode may be introduced using a barcoded primer that is used for priming the reverse transcription reaction (Fig 5E). This primer may be target specific or more generic like a barcoded random primer or a barcoded oligo(dT) primer.

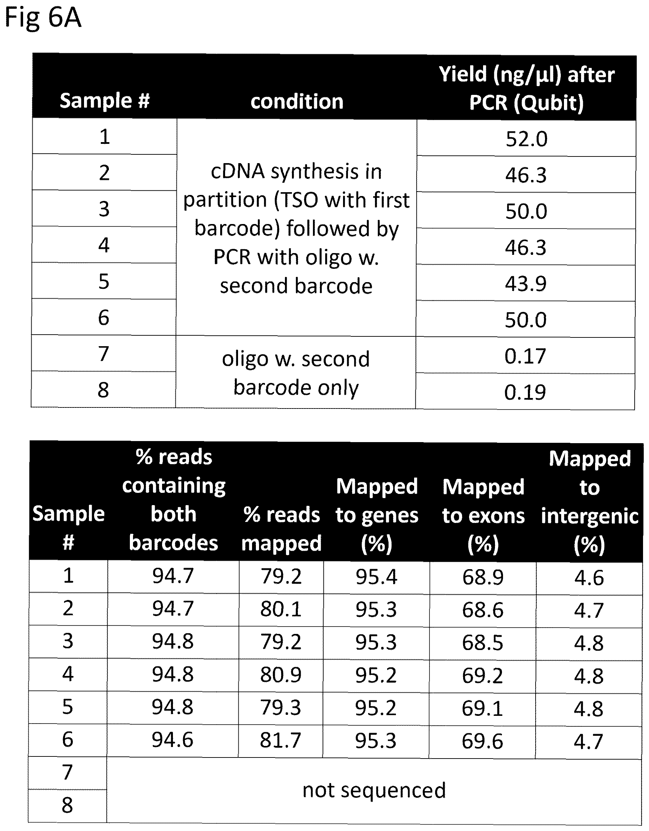

Fig. 6: **Example, barcoding of cDNA in partitions.** Example using cells as biological particles, template switching oligonucleotides containing a first barcode sequence and additional oligonucleotides containing a second barcode.
Fig. 6A, top: Yield after final amplification (after step f): successful attachment of amplified barcoded target nucleic acid to amplified barcoded oligonucleotides (high yield for samples 1 - 6 corresponding to distinct partitions [wells] compared to control samples 7 and 8).
Fig 6A, Bottom: Sequencing results for samples 1-6. The majority of reads obtained contained both barcodes (second column) and showed typical mapping statistics for cDNA libraries.

### Detailed description of the invention

In a first aspect the present invention provides a method for barcoding of nucleic acids comprising the steps:
a) Providing
   i. a plurality of biological particles comprising target nucleic acids,
   ii. a first plurality of oligonucleotides comprising a first barcode sequence, wherein each oligonucleotide of the first plurality of oligonucleotides comprises a different barcode sequence and
   iii. a second plurality of oligonucleotides comprising second barcode sequence, wherein each oligonucleotide of the second plurality of oligonucleotides comprises a different barcode sequence
b) Partitioning the plurality of biological particles such that each partition comprises a biological particle and a subset of the first and second oligonucleotides
c) Optionally releasing the target nucleic acids of the biological particles into the partition
d) Attaching to each target nucleic acid one first oligonucleotide, thereby generating barcoded target nucleic acids, wherein each (barcoded) target nucleic acid comprises a first barcode sequence, wherein this first barcode sequence is different for each target nucleic acid
e) Amplifying the second plurality of oligonucleotides and the barcoded target nucleic acids, thereby generating multiple copies of the second plurality of oligonucleotides and of the target nucleic acids
f) Attaching the second plurality of oligonucleotides to the target nucleic acids, thereby generating combinatorial barcoded target nucleic acids, wherein each (combinatorial barcoded) target nucleic acid comprises a specific combination of the first and second specific barcode sequences, wherein the specific combination of the first and second barcodes serves as partition specific barcodes

Step c) is optional, i.e. the method of the invention as defined above may comprise only steps a), b), d), e), and f), whereas an embodiment of the method of the invention as defined above comprises steps a), b), c), d), e), and f).

Said method may additionally comprise the steps
g. Breaking the partitions, thereby obtaining a mixture of combinational barcoded target nucleic acids from the partitions,
h. Sequencing the combinatorial barcoded target nucleic acids, thereby obtaining sequencing data comprising sequences of the combination of the first and second barcodes and the sequence of the target nucleic acid,
i. Using the combinational barcode for assigning the target nucleic acid to each partition.

### Step a

In the first step of the invented method a plurality of biological particles comprising target nucleic acids, a first plurality of oligonucleotides comprising a first barcode sequence, and a second plurality of oligonucleotides comprising second barcode sequence, are provided.

Biological particles according to the invention, which are provided in step a) may be selected from the group consisting of: single cells, bacterial cells, yeast cells, plant cells and viral particles, cellular organelles (nuclei, mitochondria , chloroplasts).

In one embodiment of the invention the biological particles, which are provided in step a) may be single cells. The single cells may be comprised in a sample, preferentially in a biological sample. Said single cells may be naturally occurring single cells such as circulating cells as e.g. comprised in PBMC, cord blood or bone marrow (samples). Alternatively said single cells may be derived from tissue samples such as organs of the lymphatic system or biopsies isolated from patients with disease or portions in which a disease is suspected. Said tissue samples may have been subjected to a dissociation procedure in order to break the cell association and release the cells from the tissue, thereby generating a population of single cells. Standard dissociation procedure are commonly known in the art.

Exemplary tissue samples may be blood, tonsil, lymph node, colon, pancreas, skin or any tissue of the human body.

In one embodiment of the invention said single cell suspension (or population of single cells) may be a "pure" or mixed population of cells. In other words the single cell suspension may comprise a single cell type or it may be a mixture of different cell types.

In one embodiment of the invention single cells comprised in said population of single cells may be selected from the group consisting of cells of the hematopoietic cell lineage like B cells, T cells or NK cells, or from solid tissue consisting of epithelial and mesenchymal cells.

If the single cells are a mixed population of cells, cells may be isolated or separated prior to step A) in order to obtain a specific cell population. Cell separation or isolation may be done according standard procedures or using commercially available kits, which are commonly known in the art.

In one embodiment of the invention, the biological particles may be immune cells, and the method may be used to determine the clonotype of individual cells by profiling of B-cell receptor (BCR) or T-cell receptor (TCR) mRNA or by analyzing the genomic locus of the B-cell or T-cell receptor.

In another embodiment of the invention, the biological particles may be cells derived from a tissue biopsy, and the method is used to determine the genomic integrity in individual cells, for example by analyzing known or suspected mutations implicated in cancer.

In one embodiment of the invention, the biological particles may be cellular organelles. Exemplary cell organelles are cell nucleus, mitochondria or chloroplasts. Said cellular organelles may be derived and/or may have been isolated from e.g. single cells (such as mammalian cells or plant cells) by methods known in the art prior to step a).

According to the claimed method said biological particles comprise target nucleic acids.

Said target nucleic acid may be comprised in the biological particles. In other words the target nucleic acids may be localized within the biological particles (such as single cells or cell organelles). So it has to be released from the inner of the cell (e.g. by cell lysis) prior to barcoding.

In another embodiment of the invention the target nucleic acids may be attached or bound to the surface of the cell e.g. by a nucleic acid labeled antibody (e.g. as used in the CITE-Seq approach, Stoeckius et al., 2017). Such a nucleic acid may also be referred to as a target nucleic acid. The target nucleic acid has to be released from the cell surface prior to barcoding.

Moreover in one embodiment of the invention, the target nucleic acids (DNA or RNA or chromosomes) may be the biological particles. In this embodiment of the invention, the release step (c) would not be required.

As disclosed herein, the biological particles may comprise a certain amount of target nucleic acids (nx).

Said target nucleic acids may be DNA or RNA (molecules). Said target nucleic acids may be genomic DNA, mRNA, vectors (such as plasmids, Cosmides and yeast artificial chromosomes) or viral DNA or viral RNA. In one embodiment of the invention the target nucleic acids may be genomic DNA and mRNA. In a preferred embodiment of the invention the target nucleic acids are genomic DNA or mRNA.

Dependent on the application, the target nucleic acid and/or the biological particle to be analyzed may vary. Exemplary applications are: gene expression profiling or mutation analysis of whole transcriptomes or subsets thereof, which have been comprised in a single cell (biological particle). In such an application the preferred target nucleic acid may be mRNA. Another application may be the analysis of copy number variation or mutation analysis of genomic DNA (as target nucleic acids). Another example is the analysis of (cell) surface markers. In such an example the target nucleic acids may be oligonucleotides bound to binders like antibodies.

The key elements of the current invention are the first and second plurality of oligonucleotides.

The first and second plurality of oligonucleotides may be double or single stranded, preferentially single stranded.

Each plurality of oligonucleotides comprises at least one barcode sequence. Each of the barcode sequences comprised in the oligonucleotides are different (from each other). In other words the first plurality of oligonucleotides comprises or consist of a first barcode sequence (BC1mx), wherein each oligonucleotide of the first plurality of oligonucleotides comprises a different barcode sequence (BC1mx) and; the second plurality of oligonucleotides comprises or consist of a second barcode sequence (BC2mx), wherein each oligonucleotide of the second plurality of oligonucleotides comprises a different barcode sequence (BC2mx).

In other words, the first plurality of oligonucleotides comprises the barcode sequences BC1mx, whereas the second plurality of oligonucleotides comprises the barcode sequences BC2mx. (m = specific partition; x=number of the barcode sequence)

The first plurality of oligonucleotides (comprising a first barcode sequence BC1mx) may be provided at a concentration sufficiently high to attach a barcode sequence to all target nucleic acids in step d). Therefore oligonucleotides comprised in the first plurality of oligonucleotides (comprising the barcode sequence BC1mx) may be provided in an amount nx<BC1mx.

In contrast to that, the second plurality of oligonucleotides may not be provided at a concentration sufficiently high to attach a barcode sequence to all target nucleic acids. In the amplification step e) multiple copies of the second barcode oligonucleotides are generated. Based on that, the initial concentration of the second plurality of the oligonucleotides (comprising the second barcode sequence BC2mx) does not need to be as high as the concentration of the first barcode oligonucleotides (comprising the first barcode sequence BC1mx). The concentration may be significantly lower. As a result, material and costs can be reduced.

Therefore first plurality of oligonucleotides (comprising the first barcode sequence BC1mx) may be provided in excess compared to the second plurality of oligonucleotides (comprising the second barcode sequence BC2mx). In other words the amount of oligonucleotides comprised in the first plurality of oligonucleotides (comprising the first barcode sequence BC1mx) may be provided in excess compared to the amount of oligonucleotides comprised in the second plurality of oligonucleotides (comprising the second barcode sequence BC2mx). The first plurality of oligonucleotides (comprising the first barcode sequence BC1mx) may be provided in an amount BC1mx >>> BC2mx.

In one embodiment of the invention the ratio of the first plurality of oligonucleotides (comprising the first barcode sequence BC1mx) and the second plurality of oligonucleotides (comprising the second barcode sequence BC2mx) may be (at least) 2:1 or (at least) 5:1.

The concentration of the first and second plurality of oligonucleotides is e.g. dependent on the amount of biological particles (e.g. target cells), and partitions. In one embodiment of the invention, the first plurality of oligonucleotides (comprised within one partition) may comprise at least 1, at least 5, at least 10, at least 100, at least 1000, at least 1000, at least 10.000 or at least 100.000 first oligonucleotide (molecules) per target nucleic acid (molecule). In addition to that, the second plurality of oligonucleotides (comprised within one partition) may comprise (at least) 2, (at least) 5, (at least) 10 or (at least) 100 second oligonucleotide (molecules per partition).

In one embodiment of the invention the first and/or second plurality of oligonucleotides may additionally comprise at least one primer binding sequence (Figure 1). Based on that primer binding sequences can be added to the target nucleic acids. The primer binding sequences comprised in the first and/or second plurality of oligonucleotides may be same or different, preferentially different.

In addition to that, the first and/or second plurality of oligonucleotides may additionally comprise an intermediate sequence (IM, PS2). The intermediate sequences are same or complementary in each plurality of oligonucleotides (first and second plurality of oligonucleotides). This is needed for the attachment of the second oligonucleotides to the barcoded target nucleic acids by hybridization. In addition to that, the intermediate sequence (IM, PS2) sequence may serve as additional primer binding site.

In addition to that, the first and/or second plurality of oligonucleotides may comprise an additional barcode sequence. Said additional (third) barcode sequence may be a sample specific barcode. It may be same among all partitions generated from a sample. It may be different between the partitions generated from different samples.

In one embodiment of the invention, the first and second plurality of oligonucleotides are not attached to a solid support.

The first and second pluralities of oligonucleotides are made of DNA, preferentially synthetic DNA.

In one embodiment of the invention, the oligonucleotides (first and second) may be provided (in step a)) in solution.

In another embodiment of the invention, the oligonucleotides (first and second) may be coupled to molecules or proteins such as antibodies that associate with the biological particles. In such an embodiment of the invention the oligonucleotides may be provided (in step a)) by coupling the oligonucleotide to molecules that associate with the biological particles.

### The first plurality of oligonucleotides

As disclosed herein, the first plurality of oligonucleotides comprises a barcode sequence (BC1mx). The barcode sequences are different for each oligonucleotide of the plurality of oligonucleotides. Optionally the first plurality of oligonucleotides may additionally comprise at least one primer binding sequence and/or an intermediate sequence (IM, PS2/P2). Moreover the first plurality of oligonucleotides may additionally comprise a template switching oligonucleotide (TSO, also known as template switching sequence), in order to promote template switching reactions. In order to facilitate the incorporation of the first plurality of oligonucleotides (comprising the first barcode sequence BC1mx) into the target nucleic acids, the first plurality of oligonucleotides may additionally comprise a target specific binding site (TBS), which is at least partly complementary to the target nucleic acid. Figure 1 shows examples of the structure of the first oligonucleotide.

In one embodiment of the invention, the first plurality of oligonucleotides may comprise a barcode sequence (BC1mx), an intermediate sequence (IM, PS2) and a template switching oligonucleotide (TSO). It is understood that the template switching oligonucleotide (TSO) is located at the 3' end of the oligonucleotide, whereas the intermediate sequence (IM, PS2) is located at the 5'end of the oligonucleotide (Figure 1B). Accordingly the barcode sequence (BC1mx) is positioned in between these sequences. Additional sequences encoding e.g. additional barcode or primer sequences may also be positioned in between the intermediate sequence (IM, PS2) and the template switching oligonucleotide (TSO) sequence. In a preferred embodiment of the invention, the first plurality of oligonucleotides may have or may comprise the structure (5'-3'): intermediate sequence (IM, PS2) - barcode sequence (BC1mx)- template switching oligonucleotide (TSO).

In another embodiment of the invention, the first plurality of oligonucleotides may comprise a barcode sequence (BC1mx) and primer binding sequence (PS3). It is understood that the primer binding sequence (PS3) is located at the 5' end of the oligonucleotide, whereas the first barcode sequence (BC1mx) is positioned 3' of the primer binding sequence (PS3). Moreover additional sequences encoding for e.g. additional barcode or primer binding sequences may also be positioned 5' of the primer binding sequence (PS3). In a preferred embodiment of the invention the first plurality of oligonucleotides may have or may comprise the structure (5'-3') primer binding sequence (PS3) - barcode sequence (BC1mx).

In yet another embodiment of the invention, the first plurality of oligonucleotides may comprise a barcode sequence (BC1mx), an intermediate sequence (IM, PS2) and target specific binding site (TSB). It is understood that the target specific binding site is located at the 3' end of the oligonucleotide, whereas the intermediate sequence (IM, PS2) is located at the 5'end of the oligonucleotide. Accordingly the barcode sequence (BC1mx) is positioned in between these sequences. It is understood that additional sequences encoding for e.g. additional barcode or primer binding sequences may also be positioned in between the target specific binding site (TSB) and the intermediate sequence (IM, PS2). In a preferred embodiment of the invention the first plurality of oligonucleotides may have or may comprise the structure (5'-3') intermediate sequence (IM, PS2) - barcode sequence (BC1mx)- target specific binding site (TSB) (Figure 1A).

The second plurality of oligonucleotides comprises a second barcode sequence (BC2mx), as disclosed herein. The barcode sequences are different for each oligonucleotide of the second plurality of oligonucleotides. In addition to that the (second) barcode sequences (BC2mx) comprised in the second plurality of oligonucleotides may be different from the (first) barcode sequences (BC1mx) comprised in the first plurality of oligonucleotides.

Moreover the second plurality of oligonucleotides may additionally comprise at least one primer binding sequence (PS1). Said at least one primer binding sequence may be located 5' of the barcode sequence (BC2mx). Based on that, the structure of the first plurality of oligonucleotides may be or may comprise (5'-3'): Primer binding sequence (such as PS1) - barcode sequence (BC2mx).

In addition to that, the second plurality of oligonucleotides may additionally comprise an intermediate sequence (IM, PS2). All intermediate sequences are same in the first plurality of oligonucleotides and all intermediate sequences comprised in the second plurality of oligonucleotides are also same. In addition to that, said intermediate sequence (IM, PS2) may be (at least partly) complementary to the intermediate sequence (IM, PS2) comprised in the first plurality of oligonucleotides. In one embodiment of the invention, this intermediate sequence is utilized for the attachment of the second oligonucleotides to the barcoded target nucleic acids by hybridization (step f). In addition to that, the intermediate sequence (IM, PS2) may serve as additional primer binding site/sequence. It is understood that the intermediate sequence (IM, PS2) is positioned at the 3' end of the oligonucleotide, whereas the primer binding sequence (PS1) is positioned at the 5' end of the oligonucleotide. Accordingly the barcode sequence

(BC2mx) is positioned in between these sequences. It is understood that the oligonucleotide may also have the reverse complement orientation (i.e. in 5'-3' orientation: IM - BC2mx - PS2), or that the oligonucleotide is double-stranded (partially or over the full length). It is understood that additional sequences encoding for e.g. additional barcode or primer binding sites may also be positioned in between the primer binding sequence (PS1) and the intermediate sequence (IM, PS2) sequence. In this embodiment of the invention, the second plurality of oligonucleotides may have or may comprise the structure (5'-3'): intermediate sequence (IM, PS2) - barcode sequence (BC1mx) - primer binding sequence (PS1).

In one embodiment of the invention the second plurality of oligonucleotides may not comprise an intermediate sequence as disclosed herein. In that embodiment of the invention said second plurality of oligonucleotides may comprise another primer binding sequence (PS2). The primer binding sequence (PS2) are same in each plurality of the second oligonucleotides. Said primer binding sequence (PS2) may serve as additional primer binding site. It is understood that the primer binding sequence (PS2) is positioned at the 3' end of the oligonucleotide, whereas the primer binding sequence (PS1) is positioned at the 5' end of the oligonucleotide or vice versa. Accordingly the barcode sequence (BC2mx) is positioned in between these sequences. It is understood that additional sequences encoding for e.g. additional barcode or primer binding sites may also be positioned in between the primer binding sequence (PS2) and the primer binding sequence (PS1). In this embodiment of the invention, the second plurality of oligonucleotides may have or may comprise the structure (5'-3'): primer binding sequence (PS1) - barcode sequence (BC2mx) - primer binding sequence (PS2).

In one embodiment of the invention the second plurality of oligonucleotides may comprise an additional (third) barcode sequence (BC3). This additional (third) barcode sequence BC3 may be same for all second oligonucleotides comprised in the second plurality of oligonucleotides. Such a barcode could serve e.g. as sample specific barcode. This additional (third) barcode sequence may be positioned between the primer binding sequence (PS1) and the barcode sequence (BC2mx) or between the barcode sequence (BC2mx) and the intermediate sequence (IM, PS2). Therefore, in one embodiment of the invention, the second plurality of oligonucleotides may have or may comprise the structure (5'-3'): primer binding sequence (PS1) - additional (third) barcode sequence (BC3) - barcode sequence (BC2mx) - intermediate sequence (IM, PS2). In another embodiment of the invention the second plurality of oligonucleotides may have or may comprise the structure (5'-3'): primer binding sequence (PS1) - barcode sequence (BC2mx) - additional (third) barcode sequence (BC3) - intermediate sequence (IM, PS2).

### Step b) Partitioning

In step b) the plurality of biological particles are partitioned such that each partition comprises a biological particle and a subset of the first and second oligonucleotides. Based on that a plurality of partitions is generated.

In other words, each partition from the plurality of partitions comprises a biological particle, (comprising target nucleic acids nx), a subset of the first oligonucleotides comprising the barcode sequence BC1mx and the second oligonucleotides comprising the barcode sequence BC2mx. As an example a first partition may comprise a subset of the first (BC11x) and second (BC21x) barcode sequences (comprised in the first and second plurality of oligonucleotides), whereas a second partition may comprise another subset of the first (BC12x) and second (BC22x) barcode sequence (comprised in the first and second plurality of oligonucleotides) and a third partition may comprise another subset of the first (BC13x) and second (BC23x) barcode sequences (comprised in the first and second plurality of oligonucleotides) and so on.

Standard procedures for partitioning are known in the art. Example protocols for partitioning cells using droplets or microwells are the Drop-seq approach (Macosco et al., 2015) or the Smart-Seq approach (Ramskold et al., 2012), respectively. In addition to that commercially available devices like the 10x Genomics Chromium Controller (10x Genomics, Pleasanton, CA, USA) can be used for partitioning of the components into droplets.

Depending on the method for generating partitions, some partitions may be empty or contain multiple biological particles. This is considered to be in the margin of error of the method. It is, however, desired that the number of partitions with multiple biological particles is kept as small as possible. Assigning of biological particles may be statistical or by an active process. As defined herein, the formulation "each partition comprises a biological particle" refers to a statistical distribution of the biological particles in the partition (within the margin of error of the methods).

In one embodiment of the invention, among the plurality of the partitions more than 1%, more than 10%, preferentially more than 50 % of the partitions have the given composition.

In one embodiment of the invention, a partition may be a droplet comprising a biological particle, a subset of the first and second plurality of oligonucleotides. Droplets may be generated according to protocols known in the art.

In another embodiment of the invention, a partition may be a microwell comprised e.g. in a plate. A microwell may comprise a biological particle, a subset of the first and second oligonucleotides.

It is understood that the partition further comprises reagents needed for the reactions of the method and its embodiments. The partition may therefore comprise additional primer sets, a reaction buffer, nucleotides (dNTPs) and enzymes such as enzymes for amplification, ligation, fragmentation.

### (optional) Step c) releasing of target nucleic acids

Step c) is the release of the target nucleic acids from the biological particles into the partition. It is understood that this step is performed within the partition. Methods for such a release are known in the art.

In one embodiment of the invention the biological particles may be single cells and the release step may be a cell lysis step. As a consequence the target nucleic acid molecules are released into the partition.

Depending on the biological particle and the target nucleic acid, also non target nucleic acids may be released into the partition. Based on that, a mixture comprising target and non-target nucleic acids is obtained within the partition.

Lysis of the cells may be done enzymatically and/or chemically using specific buffer conditions. Several methods and compositions are known in the art. An example for a lysis buffer compatible with subsequent hybridization of RNA (especially mRNA) to capture oligonucleotides is the Lysis/Binding Buffer for Dynabeads^{™} mRNA Purification Kits (Cat. No. A33562, ThermoFisher Scientific, Waltham, MA, USA).

### Step d) attaching the first oligonucleotide to the target nucleic acid

Step d) of the invented method comprises a step of attaching to each target nucleic acid (such as nx) one first oligonucleotide (comprising a barcode sequence BC1mx), thereby generating barcoded target nucleic acids, wherein each target nucleic acid comprises a first barcode sequence (nx-BC1mx, does not represent a specific construct), wherein this first barcode sequence is different for each target nucleic acid.

As an example in one partition, a first target nucleic acid n1 may comprise BC1.1.1, a second target nucleic acid n2 may comprise BC1.1.2, a third target nucleic acid n3 may comprise BC1.1.3 and so on. Based on that the barcoded target nucleic acids may be: n1-BC1.1.1, n2-BC1.1.2, n3-BC1.1.3 .... nx-BC1.1.x. In another partition, a first target nucleic acid n1 may comprise BC1.2.1, a second target nucleic acids n2 may comprise BC1.2.2, a third target nucleic acid may comprise BC1.2.3 and so on. Based on that the barcoded target nucleic acids may be: n1-BC1.2.1, n2-BC1.2.2, n3-BC1.2.3 .... nx-BC1.2.x.

In one embodiment of the invention, attaching said oligonucleotides may be done by ligation using ligases, hybridization followed by primer extension, template switching during the reverse transcription reaction, or other reactions well known in the art. Common examples for such ligases are e.g. T4 DNA ligase, Taq ligase or equivalent enzymes. Common examples for enzymes facilitating reverse transcription are the MMLV reverse transcriptase and its derivates. To facilitate the ligation of the oligonucleotide to double stranded target nucleic acids, the oligonucleotides as disclosed herein might be double stranded or partially double stranded.

In one embodiment of the invention, said oligonucleotide may be attached to the target nucleic by hybridization followed by primer extension. Based on that, an oligonucleotide from the first plurality of oligonucleotides may hybridize to the target nucleic acid and serve as a primer. Such a primer can then be used as starting point for an extension and/or amplification reaction. One example for this embodiment is the incorporation of the barcoded nucleic acid during cDNA synthesis in which the barcoded nucleic acid serves as primer for the cDNA synthesis. Another example is the synthesis of a reverse complimentary nucleic acid using a single-stranded DNA molecule as template. This synthesis may be linear or part of an polymerase chain reaction or other amplification reaction like whole genome amplification using Phi29 polymerase.

In another embodiment of the invention, the oligonucleotide (from the first plurality of oligonucleotides) may serve as primer during an amplification reaction. In one embodiment of the invention said oligonucleotide may serve as a primer in early cycles of an amplification reaction (such as a polymerase chain reaction), thereby generating barcoded target nucleic acids. In later cycles, the barcoded target nucleic acids (generated in the early cycles of amplification, may be amplified using a separate set of primers.

In another embodiment of the invention, the first oligonucleotide is attached by reverse transcription and template switching. In this embodiment of the invention the target nucleic acid may be mRNA and the first oligonucleotide additionally comprises a template switching sequence (TSO). Based on that, the barcode is attached to the target nucleic acid (or a copy thereof) by a reverse transcription reaction with template switching, wherein the barcode oligonucleotides serve as template switching oligonucleotides. The newly synthetized nucleic acid is referred to as "cDNA". Moreover an additional primer such as an oligo dt primer to innate cDNA synthesis may be needed.

Techniques and conditions for reverse transcription reaction with template switching are well known in the art. Examples for protocols for reverse transcription with template switching can be found in Zhu et al, 2001 and Wellenreuther et al., 2004. Key elements are a specific polymerase and a template switching oligonucleotide. Commonly used polymerases are the Moloney Murine Leukemia Virus Reverse Transcriptase (MMLV-RT) and its derivates, or Thermostable group II intron reverse transcriptase (TIGRT).

Based on that target nucleic acids comprising the first oligonucleotide are generated, in other words barcoded target nucleic acids are generated.

### Step e) Amplification of second plurality of oligonucleotides and the barcoded target nucleic acids

Step e) encompasses two amplification steps, first the amplification of the second plurality of oligonucleotides and second the amplification of the barcoded target nucleic acids. It is understood that the order of these amplification may be reverse, and the amplification may also occur simultaneously.

In the first amplification step, the second plurality of oligonucleotides is amplified. In this step the second plurality of oligonucleotides serve as a template. As disclosed herein the oligonucleotides comprise at least one primer binding sequence (e.g. PS1 and/or PS2).

In a preferred embodiment of the invention, the oligonucleotide comprises two primer binding sequences flanking the barcode sequence, or a primer binding sequence and a intermediate sequence.

Amplification primer can bind to the specific sequences and initiate the amplification process. To that end multiple copies of the second oligonucleotides are generated. Each copy of an oligonucleotide comprises the same barcode sequence. The reaction is limited by the amount of amplification primer. For this amplification reaction additional primer may be provided (comprised in the partition).

There follows the second amplification reaction in which the barcoded target nucleic acid (from step f) is amplified. In this amplification reaction the barcoded target nucleic acid serves as template. For the amplification reaction additional primer may be provided (comprised in the partition).

### Step f) attachment of the second barcode

Step f) comprises the attachment of the second plurality of oligonucleotides to the target nucleic acids. To that end combinatorial barcoded target nucleic acids are generated. Each combinatorial barcoded target nucleic acid comprises a specific combination of the first and second specific barcode sequences. This specific combination of the first and second barcodes serves as partition specific barcode. It has to be noted, that there are multiple different partition specific barcodes, i.e. not one single partition specific barcode like in prior art methods (for example the 10x Genomics Chromium system).

Said second plurality of oligonucleotides may be attached by hybridization, extension, amplification or ligation.

In a preferred embodiment of the invention the second plurality of oligonucleotides may be attached by hybridization, followed by an extension reaction. It is understood that the target nucleic acids are present as single stranded nucleic acids during the hybridization step (or partially single stranded in the region in which the hybridization occurs), as well as the second plurality of oligonucleotides. In this embodiment of the invention the second plurality of oligonucleotides may comprise a sequence (e.g. an IM sequence) which is complementary to a sequence (IM sequence) in the barcoded target nucleic acid generated in step d). The oligonucleotides bind complementary to these sites (Figure 5). There is no complete overlap between the barcoded target nucleic acids and the oligonucleotides. There follows an extension reaction in order to generate a complementary double stranded nucleic acid. Here the target nucleic acid serves as a template and the oligonucleotide serves as a primer and vice versa.

As a consequence, double stranded target nucleic acids are generated comprising a first barcode sequence (comprised in the first plurality of oligonucleotides) and a second barcode sequence (comprised in the second plurality of oligonucleotides).

In another embodiment of the invention the second plurality of oligonucleotides may be attached to the barcoded target nucleic acids by ligation. Common examples for such ligases are e.g. T4 DNA ligase, Taq ligase or equivalent enzymes.

Said oligonucleotides may be ligated to single or double stranded target nucleic acids. Consequently the second plurality of oligonucleotides may be single or double stranded.

Independent of the attachment method, a combinatorial barcode is generated which comprises a specific combination of first and second oligonucleotides (comprising the barcode sequences BC1mx and BC2mx).

### Step g) breaking the partitions

Optionally the method as claimed may additionally comprise the steps g-i). Step g is a step of breaking the partitions, thereby obtaining a mixture of combinational barcoded target nucleic acids from different partitions. Disruption of the partitions may be done chemically using specific buffer conditions.

Optionally after the disruption of the partitions the target nucleic acids may be separated from the non-target nucleic acids

Optionally a washing step is performed after step g) in order remove residuals of the lysis buffer and cell debris and/or other substances interfering with subsequent process steps. Buffer conditions are commonly known in the art.

### Step h) sequencing the combinatorial barcoded nucleic acids

After disrupting the partition there follows sequencing of the combinatorial barcoded target nucleic acids, thereby obtaining sequencing data comprising sequences of the combination of the first and second barcodes and the sequence of the target nucleic acid.

Sequencing may be done according to methods known in the art. For example by sequencing by synthesis using one of the Illumina sequencing platforms (e.g. MiSeq, NextSeq or NovaSeq; Illumina, San Diego, CA, USA), by semiconductor sequencing using an Ion Torrent Next-Generation Sequencer, Thermo Fisher Scientific, Waltham, MA, USA), by long read sequencing using the PacBio Revio sequencing system (PacBio, Menlo Park, CA, USA), or by nanopore sequencing using one of the Oxford Nanopore sequencing systems (MinIon, GridION, PromethION; Oxford Nanopore, Oxford, United Kingdom).

In one embodiment of the invention additional sequencing adapters or barcodes may be attached to the target nucleic acids (e.g. as disclosed in https://www.illumina.com/techniques/sequencing/ngs-library-prep/ligation.html.

### Step i) Data analysis

Step i) is a data analysis step. Such a data analysis step may be done according to methods known in the art. In one embodiment of the invention, the sequencing data obtained in step h) may be are aligned to sequence databases to determine the identity of the target nucleic acid. However the identity of the target nucleic acid may be assigned using alternative approaches.

Additionally, the sequence of the first (BC1mx) and second barcode sequence (BC2mx) are analyzed, and specific combinations of the first (BC1mx) and second barcode sequences (BC2mx) are assigned to specific partitions.

In more detail, sequencing data can be grouped or clustered according to the first (BC1mx) and second barcode sequences (BC2mx) comprised in the target nucleic acids.

This is based upon the fact that each first barcode and each second barcode was unique or specific. As a consequence, each partition will have a substantial number/amount of unique or specific first and second barcodes. Throughout this method, partition-specific combinations of first and second barcodes are generated.

This specific barcode combination (combination of first and second barcode) can be used to assess which of the first and second barcode were present in the same partition. This allows to assign the target nucleic acid (which was linked to the first barcode in step d)) to a partition.

This is based on the fact that each copy of a target nucleic acid comprised in one partition comprises the same first oligonucleotide comprising the same barcode sequence (BC1mx). So it is clear that they were in the same partition. In addition to that, some copies of the same target nucleic acid comprise the same second oligonucleotide comprising the barcode sequence (BC2mx), whereas other copies of the same target nucleic acid comprise a different second barcode oligonucleotide. However, based on the first oligonucleotide (comprising barcode sequence BC1mx) it is clear that even though they comprise different second oligonucleotides (comprising barcode sequence BC2mx), they originate from the same partition. Such an alignment is done for all sequencing data, thus for all target nucleic acids.

Based on the limited amount of second oligonucleotides (and therefore second barcode sequences BC2mx) provided in step a), there is only a limited amount of BC2mx combinations for each partition. So it can be traced back which BC2mx belongs to which partition, and therefore which target nucleic acid is derived from which partition.

In steps h) and i), a barcode correction may be employed. The majority of technologies for amplification and sequencing have an intrinsic error rate, therefore sequences with error may be obtained. To avoid that a barcode is incorrectly assigned to another barcode, a barcode correction step may be employed. Methods for barcode correction are commonly known in the art.

To minimize the likelihood of mis-assignment, the barcode sequences may not be random but a large number of different barcodes may be chosen, which have a hamming distance and/or levenstein distance of larger than one to ensure that amplification or sequencing errors do not lead to a barcode mis-assignment.

### Specific embodiments:

One aspect of the current invention is a method for barcoding of (target) nucleic acids comprising several steps.

Step a) comprises providing several components:
i. a plurality of biological particles comprising nx target nucleic acids,
ii. a first plurality of oligonucleotides comprising a first barcode sequence (BC1mx), wherein each oligonucleotide of the first plurality of oligonucleotides comprises a different barcode sequence and
iii. a second plurality of oligonucleotides comprising second barcode sequence (BC2mx), wherein each oligonucleotide of the second plurality of oligonucleotides comprises a different barcode sequence

wherein the first plurality of oligonucleotides (comprising the barcode sequence) is provided in an amount nx<BC1mx
wherein the second plurality of oligonucleotides (comprising the barcode sequence) is provided in an amount BC1mx > BC2mx

Step B comprises partitioning the plurality of biological particles such that each partition comprises a biological particle and a subset of the first and second oligonucleotides (comprising the barcode sequences BC1mx and BC2mx, respectively), thereby generating multiple partitions each of which comprises a different subset of the first and second plurality of oligonucleotides. As a consequence multiple partitions are generated. Exemplary a first partition may comprise a subset of the first and second oligonucleotides (comprising the barcode sequences BC11x and BC21x, respectively), whereas a second partition may comprise a subset of the first and second oligonucleotides (comprising the barcode sequences BC12x and BC22x, respectively), a third partition may comprise a subset of the first and second oligonucleotides (comprising the barcode sequences BC13x and BC23x, respectively) and so on.
(optionally) Step c) comprises releasing the target nucleic acids of the biological particles into the partition

Step d) comprises attaching to each target nucleic acid one first oligonucleotide, thereby generating barcoded target nucleic acids, wherein each target nucleic acid comprises a first barcode sequence (nx-BC11x), wherein this first barcode sequence is different for each target nucleic acid

Afterwards (step e) the second plurality of oligonucleotides (comprising the barcode sequence BC2mx) and the barcoded target nucleic acids are amplified, thereby generating multiple copies of the second plurality of oligonucleotides and of the barcoded target nucleic acids. In this step multiple copies of the second plurality of oligonucleotides (BC2mx'-x') are generated. Exemplary after this step a first partition comprises multiple copies of the second plurality of oligonucleotides e.g. BC21x; BC21x' , BC21x''; and multiple copies of the barcoded target nucleic acid (nx-BC11x); (nx'-BC11x'); (nx''-BC11x'').

There follows step f) in which the second plurality of oligonucleotides is attached to the target nucleic acids, thereby generating combinatorial barcoded target nucleic acids, wherein each target nucleic acid comprises a specific combination of the first and second specific barcode sequences, wherein the specific combination of the first and second barcode sequences serves as partition specific barcodes. The attachment of the second plurality of oligonucleotides results in combinatorial barcoded target nucleic acids for example: nx-BC11x - BC21x. Based on the different amounts of first and second oligonucleotides provided in step a) statistically each target nucleic acids comprises a specific barcode of the first plurality of oligonucleotides. In addition to that each copy n' of the target nucleic acid generated in step e) comprises the same specific barcode sequence as the "original" target nucleic acid.

For example if a partition comprised three target nucleic acids N1, N2 and N3, after step e) they would comprise BC1.1.1, BC1.1.2 and BC1.1.3. In Step e) multiple copies of these barcoded nucleic acids are generated. For example:
- N1-BC1.1.1, N1'-BC1.1.1; N1"-BC1.1.1; N1-BC1.1.1''' ...
- N2-BC1.1.2, N2'-BC1.1.2; N2"-BC1.1.2; N2‴-BC1.1.2 ....
- N3-BC1.1.3; N3'-BC1.1.3; N3"-BC1.1.3, N3"'-BC1.1.3...
In addition to that in step e) multiple copies of the second plurality of oligonucleotides (comprising barcode sequences BC2mx) are generated: For example:
- BC2.1.1.1; BC2.1.1.1'; BC2.1.1.1'', BC2.1.1.1‴
- BC2.1.1.2; BC2.1.1.2'; BC2.1.1.2'', BC2.1.1.2‴
- BC2.1.1.3; BC2.1.1.3'; BC2.1.1.3'', BC2.1.1.3‴

The attachment of the second plurality of oligonucleotides results in combinatorial barcoded nucleic acids. Statistically each copy of a target nucleic acid comprises same or different BC2mx sequences

Some copies of a target nucleic acids comprise different BC2mx sequences, while some copies of the same target nucleic acid comprise same BC2mx sequences. Thereby generating specific barcode combinations for each partition / each pool of target nucleic acids. Each pool/ copies of target nucleic acids will comprise similar combinations of / with the second BC2mx. Based on the specific combinations of the barcode sequences (BC1mx and BC2mx) comprised in each pool / copied pool of target nucleic acids, it can be traced back from which partition the target nucleic acid was derived from / which target nucleic acids where in the same partition.

There follows an analysis step which based on clustering sequencing data according to the first barcode (BCmx) sequence. Every target nucleic acid comprising the same barcode sequence (BC1mx) (which was comprised in the first plurality of oligonucleotides) is derived from the initial target nucleic acid or is a copy thereof. These copies where in one partition.

Additionally the sequencing data may be clustered according to the (second) barcode sequence (which was comprised in the second plurality of oligonucleotides). It is based on the assumption that every target nucleic acid comprising the same first barcode sequence (BC1mx) is derived from the initial target nucleic acid or is a copy thereof. Consequently every target nucleic acid comprising the same first barcode sequence (BC1mx) but a different second barcode is derived from the initial target nucleic acid / or is a copy thereof, consequently these second barcode sequences (BC2mx) are the barcodes of the partition. Consequently other target nucleic acids comprised in the same partition comprise similar second barcode sequences (BC2mx), which can be identified / assigned by using the first barcode sequence (BC1mx).

### Variant 1: Attachment of second oligonucleotides by hybridization in step f)

In one variant of the invention, the second plurality of oligonucleotides may be attached to the target nucleic acids by hybridization.

Prerequisite is the introduction of the intermediate sequence (IM, PS2) which is complementary to one part of the second plurality of oligonucleotides.

The intermediate sequence ((IM, PS2)) may be introduced to the target nucleic acid using different methods and different primer combinations, which will be described in the following embodiments, depending on the target nucleic acid and primer construct. The composition or structure of the first plurality of oligonucleotides (comprising the barcode sequence BC1mx) may differ depending on the method and target nucleic acid.

In contrast to that the second plurality of oligonucleotides (comprising the barcode sequence BC2mx) provided in step a) will be same for all embodiments. Said second plurality of oligonucleotides may comprise a primer binding sequence, an intermediate sequence and a barcode sequence. Preferentially the second plurality of oligonucleotides may have or may comprise the structure (5'-3'): intermediate sequence (IM, PS2) - barcode (BC2mx) - primer binding sequence (PS1).

In a first embodiment of this variant of the invention, the target nucleic acid may be mRNA (e.g. comprised in single cells) and the first plurality of oligonucleotides may comprise a first barcode sequence (BC1mx), an intermediate sequence (IM, PS2) and a template switching oligonucleotide (TSO), wherein each oligonucleotide of the first plurality of oligonucleotides comprises a different barcode sequence (BC1mx). Preferentially said first plurality of oligonucleotides may have or may comprise the structure (5'-3') intermediate sequence (IM, PS2) - barcode (BC1mx) - template switching oligonucleotide (TSO).

Moreover the following components may be additionally provided in step a)
- Primer for cDNA synthesis and attachment of the first plurality of oligonucleotides . These primer comprise either an oligo dt sequence (at the 3'end) to bind to the poly A tail of the mRNA; or a specific primer sequence (at the 3'end), which is complementary to a sequence within the target nucleic acid (mRNA). Moreover the primer may comprise an additional primer binding sequence (PS3), which is not complementary to the target mRNA, but which is incorporated during cDNA synthesis.
- Primer pairs (forward IM; PS2 and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM/PS2 and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

In step d) the first plurality of oligonucleotides (comprising barcode sequence BC1mx) is attached to the target mRNA by template switching. More specifically, in this process the primer for cDNA synthesis (oligo dt or specific for a sequence within the target mRNA) bind to the target mRNA and serves as a starting point for reverse transcription. The reverse transcriptase with template switching activity reverse transcribes the target mRNA into cDNA and the first plurality of oligonucleotides is attached to the target cDNA by template switching. As a result, the target cDNA comprises said first barcode sequence (BC1mx). In addition to that, the target cDNA comprises the primer binding site PS3 and IM (PS2) at the 3' and 5' end of each target nucleic acid, respectively. Preferentially the target nucleic acid has the structure (5'-3'): intermediate sequence (IM, PS2) - barcode sequence (BC1mx) - template switching oligonucleotide (TSO) - target nucleic acid (sequence) - primer binding sequence (PS3).
In a second embodiment of this variant of the invention the target nucleic acid may be mRNA (e.g. from single cells) and the first plurality of oligonucleotides may comprise a barcode sequence (BC1mx), primer binding sequence (PS3) and a target specific binding site (TSB, which serves as primer sequence for cDNA synthesis). Said target specific binding site may comprise either an oligo dt sequence (at the 3'end) to bind to the poly A tail of the mRNA; or a specific sequence (at the 3'end), which is complementary to a sequence within the target nucleic acid (mRNA). Preferentially, the first plurality of oligonucleotides may have or may comprise the structure (5'-3'): target specific binding site (TSB) - barcode sequence (BC1mx)- - primer binding sequence(PS3).

Moreover the following components may be additionally provided in step a)
- Template switching oligonucleotide (TSO) comprising an intermediate sequence (IM, PS2), wherein the TSO is at the 3' end and the intermediate sequence is at the 5' end of the oligonucleotide.
- Primer pairs (forward IM/PS2 and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM/PS2 and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

Step d) is attaching to each target mRNA one first oligonucleotide (comprising the barcode sequence BC1mx), thereby generating barcoded target cDNA (target nucleic acids), wherein each cDNA comprises a first barcode sequence (BC1mx), wherein this first barcode sequence is different for each target cDNA. In this embodiment of the invention, the first plurality of oligonucleotides are attached to the target cDNA by template switching. More specifically, in this process the first plurality of oligonucleotides comprises a target specific binding site (TSB), which serves primer sequence for cDNA synthesis (oligo dt or specific for a sequence within the target mRNA). It binds to the mRNA and serves as a starting point for reverse transcription. The reverse transcriptase with template switching activity reverse transcribes the target mRNA into cDNA and the template switching oligonucleotide is attached to the target cDNA by template switching. As a result the target cDNA comprises said first barcode sequence (BC1mx) . In addition to that, the target cDNA comprises the primer binding site IM/PS2 and PS3 at the 3' and 5' end of the target nucleic acid respectively. Preferentially the target nucleic acid has the structure (5'-3'): intermediate sequence (IM, PS2) -template switching oligonucleotide (TSO) - target nucleic acid (sequence) - barcode sequence (BC1mx) - primer binding sequence (PS3).

In a third embodiment of this variant of the invention, the target nucleic acid may be mRNA (e.g. from single cells) and the first plurality of oligonucleotides provided in step a) may comprise a barcode sequence (BC1mx), an intermediate sequence (IM, PS2) and a target specific binding site (TSB) as disclosed herein. Said target specific binding site sequence may be specific for a sequence in the target nucleic acid. Preferentially said first plurality of oligonucleotides (BC1mx) may have or may comprise the structure (5'-3') intermediate sequence (IM, PS2) - barcode (BC1mx) - target specific binding site (TSB).

Moreover the following components may be additionally provided in step a )
- Primer for cDNA synthesis and attachment of the first plurality of oligonucleotides comprising the barcode sequence (BC1mx). These primer comprise either an oligo dt sequence (at the 3'end) to bind to the poly A tail of the mRNA; or a specific primer sequence (at the 3' end), which is complementary to a sequence within the target nucleic acid (mRNA). Moreover the primer may comprise an additional primer binding sequence (PS3), which is not complementary to the target mRNA, but which is incorporated during cDNA synthesis.
- Primer pairs (forward IM/PS2 and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM/PS2 and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

There follows in step d) attaching to each target mRNA one first oligonucleotide comprising said barcode sequence (BC1mx), thereby generating barcoded target cDNA (target nucleic acids), wherein each cDNA comprises a first barcode sequence (BC1mx), wherein this first barcode sequence is different for each target cDNA. In this embodiment, the first plurality of oligonucleotides are attached to the target cDNA in an amplification reaction. In this process the primer for cDNA synthesis (oligo dt or specific for a sequence within the target mRNA) binds to the target mRNA and serves as a starting point for reverse transcription. The reverse transcriptase reverse transcribes the target mRNA into cDNA. The strands are separated and the first plurality of oligonucleotides (comprising barcode sequence (BC1mx)) binds to the target cDNA (via TSB) and serves as a primer and therefore as a starting point for a second strand synthesis. This then leads to incorporation of the first plurality of barcode oligonucleotides (comprising barcode sequence (BC1mx)) into the target cDNA. As a result the target cDNA comprises said first barcode sequence (BC1mx ). In addition to that, the target cDNA comprises the primer binding site IM/PS2 and PS3 at the 3' and 5' end of the target nucleic acid respectively. Preferentially the target nucleic acid has the structure (5'-3'): intermediate sequence (IM, PS2) - barcode sequence (BC1mx) - target nucleic acid (sequence) - primer binding sequence (PS3).

In another embodiment of this variant of the invention the target nucleic acid may be mRNA (e.g. from single cells) and the first plurality of oligonucleotides may comprise a barcode sequence (BC1mx), a primer binding sequence (PS3) and a target specific binding site (TSB). Said target specific binding site (TSB) sequence may be an oligo dt sequence or a sequence complementary to a sequence comprised in the target nucleic acid). Preferentially, the first plurality of oligonucleotides may have or may comprise the structure (5'-3'): primer binding sequence (PS3) - first barcode sequence (BC1mx)- target specific binding site (TSB).

Moreover the following components may be additionally provided
- Primer comprise a specific sequence, which is complementary to a sequence within the target mRNA (cDNA) and an intermediate sequence (IM, PS2). The intermediate sequence is at the 5' end of the oligonucleotide.
- Primer pairs (forward IM and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

There follows step d) attaching to each target mRNA one first oligonucleotide (comprising a first barcode sequence (BC1mx)), thereby generating barcoded target cDNA (target nucleic acids), wherein each cDNA comprises a first barcode sequence (BC1mx), wherein this first barcode sequence is different for each target cDNA. In this embodiment, the first plurality of oligonucleotides are attached to the target cDNA in an amplification reaction. In this process the first plurality of oligonucleotides serve as primer for cDNA synthesis (TSB, oligo dt or specific for a sequence within the target mRNA) binds to the target mRNA and serves as a starting point for reverse transcription. The reverse transcriptase reverse transcribes the target mRNA into cDNA. The strands are separated and the primer comprising the IM and the complementary sequence bind to the target cDNA and serve as a primer and therefore as a starting point for a second strand synthesis. This then leads to incorporation of the first plurality of barcode oligonucleotides (comprising the first barcode sequence (BC1mx)) into the target cDNA. As a result the target cDNA comprises said first barcode sequence (BC1mx). In addition to that, the target cDNA comprises the primer binding site IM (PS2) and PS3 at the 3' and 5' end of the target nucleic acid respectively. Preferentially the target nucleic acid has the structure (5'-3'): intermediate sequence (IM, PS2) -barcode sequence (BC1mx) - target nucleic acid (sequence) - primer binding sequence (PS3).

In another embodiment of this variant of the invention, the target nucleic acid may be genomic DNA (e.g. from single cells) and the first plurality of oligonucleotides provided in step a) may comprise a barcode sequence (BC1mx), an intermediate sequence (IM, PS2) and a target specific binding site (TSB) as disclosed herein. Said target specific binding site (TSB) may be specific for a sequence in the target nucleic acid. Preferentially said first plurality of oligonucleotides (BC1mx) may have or may comprise the structure (5'-3') intermediate sequence (IM, PS2) - barcode (BC1mx) - target specific binding site (TSB).

Moreover the following components may be additionally provided
- Primer comprise a specific primer sequence (at the 3'end), which is complementary to a sequence within the target genomic DNA. Moreover the primer may comprise an additional primer binding sequence (PS3), which is not complementary to the target sequence in the genomic DNA, but which is incorporated during attachment of the first oligonucleotides.
- Primer pairs (forward IM/PS2 and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM/PS2 and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

In step d) to each target genomic DNA (or fragments) one first oligonucleotide (comprising the first barcode sequence (BC1mx)) is attached, thereby generating barcoded target genomic DNA (or fragments), wherein each genomic DNA (or fragments) comprises a first barcode sequence, wherein this first barcode sequence (BC1mx) is different for each target genomic DNA (or fragments). In this embodiment, the first plurality of oligonucleotides (comprising the first barcode sequence (BC1mx)) are attached to the target genomic DNA (or fragments) in an amplification reaction. In this process the primer for genomic DNA (or fragments) synthesis (primer specific for a sequence within the target genomic DNA (or fragments)) binds to the target genomic DNA (or fragments) and serves as a starting point for DNA synthesis. The strands are separated and the first plurality of oligonucleotides (comprising the first barcode sequence (BC1mx)) binds to the target genomic DNA (or fragments) (via target specific binding site (TSB)) and serves as a primer and therefore as a starting point for a second strand synthesis. This then leads to incorporation of the first plurality of oligonucleotides ((comprising the first barcode sequence (BC1mx)) into the target genomic DNA (or fragments, thereof). As a result the target genomic DNA (or fragments) comprises said first barcode sequence (BC1mx) . In addition to that, the target genomic DNA (or fragments) comprises the primer binding site IM (PS2) and PS3 at the 3' and 5' end of the target nucleic acid respectively. Preferentially the target nucleic acid has the structure (5'-3'): intermediate sequence (IM, PS2) -barcode sequence (BC1mx) - target nucleic acid (sequence) - primer binding sequence (PS3).

In another embodiment of this variant of the invention the target nucleic acid is target genomic DNA (e.g. from single cells) and the first plurality of oligonucleotides comprising a barcode sequence (BC1mx), a primer binding sequence (PS3) and a target specific binding site (TSB). Said a target specific binding site (TSB) sequence may be a specific sequence complementary to a sequence comprised in the target nucleic acid. Preferentially, the first plurality of oligonucleotides may have or may comprise the structure (5'-3'): primer binding sequence (PS3) -- first barcode sequence (BC1mx)- a target specific binding site (TSB).

Moreover the following components may be additionally provided
- Primer comprise a specific sequence, which is complementary to a sequence within the target genomic DNA and an intermediate sequence (IM, PS2). The intermediate sequence is at the 5' end of the oligonucleotide/primer.
- Primer pairs (forward IM and reverse primer PS1) specific for the amplification of the second plurality oligonucleotides in step e). The forward primer (IM, PS2) may be specific for the intermediate sequence and the reverse primer may be specific for the primer binding sequence (PS1) or vice versa.
- Primer pairs (forward IM/PS2 and reverse PS3) specific for the amplification of the barcoded target nucleic acids in step e). The forward primer may be specific for the intermediate sequence (IM, PS2) and the reverse primer (PS3) is specific for the primer binding sequence (PS3) or vice versa.

There follows step d) attaching to each target genomic DNA (or target genomic DNA fragments) one first oligonucleotide, thereby generating barcoded target genomic DNA (or target genomic DNA fragments) (target nucleic acids), wherein each genomic DNA (or target genomic DNA fragments) comprises a first barcode sequence (BC1mx), wherein this first barcode sequence is different for each target genomic DNA (or target genomic DNA fragments). In this embodiment, the first plurality of oligonucleotides are attached to the target genomic DNA (or target genomic DNA fragments) in an amplification reaction. In this process the first plurality of oligonucleotides serve as primer for genomic DNA (or target genomic DNA fragments) synthesis, bind to the target genomic DNA (or target genomic DNA fragments) and serves as a starting point for nucleic acid synthesis. As a result the first barcode sequence BC1mx is incorporated into the target nucleic acid. After nucleic acid synthesis, the strands are separated and the Primer comprising a specific sequence, which is complementary to a sequence within the target genomic DNA (and an intermediate sequence (IM, PS2)) bind to the target genomic DNA (or target genomic DNA fragments) and serve as a starting point for a second strand synthesis. This then leads to incorporation of the intermediate sequence. As a result the target genomic DNA (or target genomic DNA fragments) comprises said first barcode sequence and an intermediate sequence. In addition to that, the target genomic DNA (or target genomic DNA fragments) comprises the primer binding site IM (PS2) and PS3 at the 3' and 5' end of the target nucleic acid respectively. Preferentially the target genomic DNA (or target genomic DNA fragments) has the structure (5'-3'): intermediate sequence (IM, PS2) - target nucleic acid (sequence) - barcode sequence (BC1mx) - primer binding sequence (PS3).

Independent of whether the target nucleic acids are cDNA, mRNA or genomic DNA, all target nucleic acids generated in Step d) comprise an intermediate sequence at the 5' end, which can be used for hybridization of the second plurality of barcodes.

Based on that in step e) the second plurality of oligonucleotides and the barcoded target nucleic acids are amplified, thereby generating multiple copies of the second plurality of oligonucleotides and of the barcoded target nucleic acids. More specifically, the second plurality of barcodes are amplified using primer specific for the intermediate sequence (IM, PS2) and the primer binding sequence(PS1). Moreover the barcoded target nucleic acids may be amplified using primer specific for the intermediate sequence (IM, PS2) and specific for the primer binding sequence (PS3). The amplification of both (oligonucleotides and barcoded target nucleic acids) may be done by PCR. The outcome of this process are multiple copies of the barcoded target cDNA and multiple copies of each oligonucleotide comprised in the second plurality of oligonucleotides.

There follows step f), which is attaching the second plurality of oligonucleotides to the target cDNA, thereby generating combinatorial barcoded target nucleic acids, wherein each barcoded target nucleic acids comprises a specific combination of the first (BC1mx) and second (BC2mx) specific barcode sequences, wherein the specific combination of the first and second barcodes serves as partition specific barcode. More specifically the second plurality of oligonucleotides may be attached to the barcoded target nucleic acids by hybridization. It is the complementary hybridization of the intermediate sequences (IM, PS2) comprised at the 3' end of the second plurality of oligonucleotides and at the 3' end of the target barcoded target nucleic acids. After hybridization there follows a strand extension reaction in which the target barcoded target nucleic acids and the oligonucleotide are extended by a polymerase, thereby generating double stranded target barcoded target nucleic acids. This target nucleic acid comprises two barcodes which can serve as partition specific barcode.

Optionally, the method may additionally comprise the steps breaking the partitions, thereby obtaining a mixture of combinational barcoded target nucleic acids from the partitions.

Sequencing combinatorial barcoded target nucleic acids, thereby obtaining sequencing data comprising sequences of the combination of the first and second barcodes and the sequence of the target nucleic acid. Using the combinational barcode for assigning the target nucleic acid to each partition.

### Variant 2: Attachment of second oligonucleotides by ligation

In another variant of the invention, the second plurality of oligonucleotides may be attached by ligation.

In contrast to the previous variant of the invention no introduction of an intermediate site is needed if the second plurality of oligonucleotides comprising the barcode sequence (BC2mx) is attached by ligation (to the target nucleic acids).

In this variant of the invention, the attachment and the structure of the first plurality of oligonucleotides comprising the barcode sequence (BC1mx) to the target nucleic acids may vary and may be dependent on the target nucleic acid.

In contrast to that, the second plurality of oligonucleotides provided in step a) will be same for all embodiments. Said second plurality of oligonucleotides may comprise a primer binding sequence (PS2), a primer binding sequence (PS1) and a barcode sequence (BC2mx). Preferentially the second plurality of oligonucleotides may have or may comprise the structure (5'-3'): primer binding sequence (PS2) - barcode sequence (BC2mx) -primer binding sequence (PS1).

In a first embodiment of this variant of the invention, the target nucleic acid may be mRNA (e.g. comprised in single cells) and the first plurality of oligonucleotides may comprise a first barcode sequence (BC1mx), a primer binding sequence (PS4) and a template switching oligonucleotide (TSO), wherein each oligonucleotide of the first plurality of oligonucleotides comprises a different barcode sequence. Preferentially said first plurality of oligonucleotides may have or may comprise the structure (5'-3') primer binding sequence (PS4) - barcode sequence (BC1mx) - template switching oligonucleotide (TSO).

Moreover the following components may be additionally provided in step a)
- Primer for cDNA synthesis and attachment of the first plurality of oligonucleotides. These primer comprise either an oligo dt sequence (at the 3'end) to bind to the poly A tail of the mRNA; or a specific primer sequence (at the 3'end), which is complementary to a sequence within the target nucleic acid (mRNA). Moreover the primer may comprise an additional primer binding sequence (PS3), which is not complementary to the target mRNA, but which is incorporated during cDNA synthesis.
- Primer pairs (forward PS2 and reverse primer PS1) specific for the amplification of the second plurality oligonucleotides in step e).
- Primer pairs (forward PS3 and reverse PS4) specific for the amplification of the barcoded target nucleic acids in step e).

In step d) the first plurality of oligonucleotides are attached to the target mRNA by template switching. More specifically, in this process the primer for cDNA synthesis (P1, oligo dt or specific for a sequence within the target mRNA) binds to the target mRNA and serves as a starting point for reverse transcription. The reverse transcriptase with template switching activity reverse transcribes the target mRNA into cDNA and the first plurality of oligonucleotides is attached to the target cDNA by template switching. As a result the target cDNA comprises said first barcode sequence. In addition to that, the target cDNA comprises the primer binding site PS4 and PS3 at the 3' and 5' end of the target nucleic acid respectively. Preferentially the target nucleic acid has the structure (5'-3'): primer binding sequence (PS4) - barcode sequence (BC1mx) - template switching oligonucleotide (TSO) - target nucleic acid (sequence) - primer binding sequence (PS3).

In a second embodiment of this variant of the invention the target nucleic acid may be mRNA (e.g. from single cells) and the first plurality of oligonucleotides may comprise a barcode sequence (BC1mx), primer binding sequence (PS3) and a target specific binding site (TSB, which serves as primer sequence for cDNA synthesis). Said target specific binding site may comprise either an oligo dt sequence (at the 3' end) to bind to the poly A tail of the mRNA; or a specific sequence (at the 3'end), which is complementary to a sequence within the target nucleic acid (mRNA). Preferentially, the first plurality of oligonucleotides may have or may comprise the structure (5'-3'): target specific binding site (TSB) - barcode sequence (BC1mx)- - primer binding sequence(PS3).

Moreover the following components may be additionally provided in step
- Template switching oligonucleotide (TSO) comprising a primer binding sequence (PS4), wherein the TSO is at the 3' end and the intermediate sequence is at the 5' end of the oligonucleotide.
- Primer pairs (forward PS1 and reverse primer PS2) specific for the amplification of the second plurality oligonucleotides in step e).
- Primer pairs (forward PS3 and reverse PS4) specific for the amplification of the barcoded target nucleic acids in step e).

In step d) attaching to each target mRNA one first oligonucleotide, thereby generating barcoded target cDNA (target nucleic acids), wherein each cDNA comprises a first barcode sequence, wherein this first barcode sequence (BC1mx) is different for each target cDNA. In this embodiment of the invention, the first plurality of oligonucleotides are attached to the target cDNA by template switching. More specifically, in this process the first plurality of oligonucleotides comprises a target specific binding site (TSB), which serves primer sequence for cDNA synthesis (oligo dt or specific for a sequence within the target mRNA) It binds to the mRNA and serves as a starting point for reverse transcription. The reverse transcriptase with template switching activity reverse transcribes the target mRNA into cDNA and the template switching oligonucleotide is attached to the target cDNA by template switching. As a result the target cDNA comprises said first barcode sequence (BC1mx). In addition to that, the target cDNA comprises the primer binding site PS4 and PS3 at the 3' and 5' end of the target nucleic acid respectively. Preferentially the target nucleic acid has the structure (5'-3'): primer binding sequence (PS4) -template switching oligonucleotide (TSO) - target nucleic acid (sequence) - barcode sequence (BC1mx) - primer binding sequence (PS3).

In a third embodiment of this variant of the invention the target nucleic acid may be mRNA (e.g. from single cells) and the first plurality of oligonucleotides provided in step a) may comprise a barcode sequence (BC1mx), primer binding sequence (PS4) and a target specific binding site (TSB) disclosed herein. Preferentially said first plurality of oligonucleotides may have or may comprise the structure (5'-3'): primer binding sequence (PS4) - barcode (BC) - and a target specific binding site (TSB).

Moreover the following components may be additionally provided in step a )
- Primer for cDNA synthesis and attachment of the first plurality of oligonucleotides comprising the barcode sequence (BC1mx). These primer comprise either an oligo dt sequence (at the 3'end) to bind to the poly A tail of the mRNA; or a specific primer sequence (at the 3' end), which is complementary to a sequence within the target nucleic acid (mRNA). Moreover the primer may comprise an additional primer binding sequence (PS3), which is not complementary to the target mRNA, but which is incorporated during cDNA synthesis.
- Primer pairs (forward PS1 and reverse primer PS2) specific for the amplification of the second plurality oligonucleotides in step e). The forward primer (PS1) may be specific for the primer binding sequence (PS1) and the reverse primer may be specific for the primer binding sequence (PS2) or vice versa.
- Primer pairs (forward PS4 and reverse PS3) specific for the amplification of the barcoded target nucleic acids in step e). The forward primer may be specific for the primer binding sequence (PS4) and the reverse primer (PS3) is specific for the primer binding sequence (PS3) or vice versa.

There follows in step d) attaching to each target mRNA one first oligonucleotide, thereby generating barcoded target cDNA (target nucleic acids), wherein each cDNA comprises a first barcode sequence (BC1mx), wherein this first barcode sequence is different for each target cDNA. In this embodiment, the first plurality of oligonucleotides are attached to the target cDNA during cDNA synthesis. In this process the primer for cDNA synthesis (oligo dt or specific for a sequence within the target mRNA) binds to the target mRNA and serves as a starting point for reverse transcription. The reverse transcriptase reverse transcribes the target mRNA into cDNA. The strands are separated and the first plurality of oligonucleotides binds to the target cDNA and serves as a primer and therefore as a starting point for a second strand synthesis. This then leads to incorporation of the first plurality of barcode oligonucleotides into the target cDNA. As a result the target cDNA comprises said first barcode sequence (BC1mx) (Figure 1A). In addition to that, the target cDNA comprises the primer binding site PS3 and PS4 at the 3' and 5' end of the target nucleic acid respectively. Preferentially the target nucleic acid has the structure (5'-3'): primer binding sequence (PS4) - barcode sequence (BC1mx) - target nucleic acid (sequence) - primer binding sequence (PS3).

In another embodiment of this variant of the invention the target nucleic acid may be mRNA (e.g. from single cells) and the first plurality of oligonucleotides may comprise a barcode sequence (BC1mx), primer binding sequence(PS3) ) and a target specific binding site (TSB). Said target specific binding site (TSB) sequence may be an oligo dt sequence or a sequence complementary to a sequence comprised in the target nucleic acid. Preferentially, the first plurality of oligonucleotides may have or may comprise the structure (5'-3'): primer binding sequence (PS3) - first barcode sequence (BC1mx)- target specific binding site (TSB).

Moreover the following components may be additionally provided
- Primer comprise a specific sequence, which is complementary to a sequence within the target mRNA (cDNA) and an additional primer binding site (PS4). PS4 is at the 5' end of the oligonucleotide.
- Primer pairs (forward PS1 and reverse primer PS2) specific for the amplification of the second plurality oligonucleotides in step e). The forward primer (PS1) may be specific for primer binding sequence 2 and the reverse primer may be specific for the primer binding sequence (PS2) or vice versa.
- Primer pairs (forward PS4 and reverse PS3) specific for the amplification of the barcoded target nucleic acids in step e). The forward primer may be specific for the primer binding sequence (PS4) and the reverse primer (PS3) is specific for the primer binding sequence (PS3) or vice versa.

There follows step d) attaching to each target mRNA one first oligonucleotide (comprising a first barcode sequence (BC1mx)), thereby generating barcoded target cDNA (target nucleic acids), wherein each cDNA comprises a first barcode sequence (BC1mx), wherein this first barcode sequence is different for each target cDNA. In this embodiment, the first plurality of oligonucleotides are attached to the target cDNA in an amplification reaction. In this process the first plurality of oligonucleotides serve as primer for cDNA synthesis (TSB, oligo dt or specific for a sequence within the target mRNA) binds to the target mRNA and serves as a starting point for reverse transcription. The reverse transcriptase reverse transcribes the target mRNA into cDNA. The strands are separated and the primer comprising the IM and the complementary sequence bind to the target cDNA and serve as a primer and therefore as a starting point for a second strand synthesis. This then leads to incorporation of the first plurality of barcode oligonucleotides (comprising the first barcode sequence (BC1mx)) into the target cDNA. As a result the target cDNA comprises said first barcode sequence (BC1mx) (Figure 1A). In addition to that, the target cDNA comprises the primer binding site PS4 and PS3 at the 3' and 5' end of the target nucleic acid respectively. Preferentially the target nucleic acid has the structure (5'-3'): primer binding sequence (PS4) -barcode sequence (BC1mx) - target nucleic acid (sequence) - primer binding sequence (PS3).

In another embodiment of this variant of the invention the target nucleic acid may be genomic DNA (e.g. from single cells) and the first plurality of oligonucleotides provided in step a) may comprise a barcode sequence (BC1mx), target specific binding site (TSB) and a primer binding sequence(PS4) disclosed herein. Said target specific binding site (TSB) may be specific for a sequence in the target nucleic acid. Preferentially said first plurality of oligonucleotides may have or may comprise the structure (5'-3'): primer binding sequence (PS4) - barcode (BC) - target specific binding site (TSB).

Moreover the following components may be additionally provided in step a )
a. Primer comprising a specific primer sequence (at the 3'end), which is complementary to a sequence within the target genomic DNA. Moreover the primer may comprise an additional primer binding sequence (PS3), which is not complementary to the target sequence in the genomic DNA, but which is incorporated during attachment of the first oligonucleotides.
b. Primer pairs (forward PS1 and reverse primer PS2) specific for the amplification of the second plurality oligonucleotides in step e). The forward primer (PS1) may be specific for the primer binding sequence (PS1) and the reverse primer may be specific for the primer binding sequence (PS2) or vice versa.
c. Primer pairs (forward PS4 and reverse PS3) specific for the amplification of the barcoded target nucleic acids in step e). The forward primer may be specific for the primer binding sequence (PS4) and the reverse primer (PS3) is specific for the primer binding sequence (PS3) or vice versa.

In step d) to each target genomic DNA (or fragments) one first oligonucleotide (comprising the first barcode sequence (BC1mx)) is attached, thereby generating barcoded target genomic DNA (or fragments), wherein each genomic DNA (or fragments) comprises a first barcode sequence, wherein this first barcode sequence (BC1mx) is different for each target genomic DNA (or fragments). In this embodiment, the first plurality of oligonucleotides (comprising the first barcode sequence (BC1mx)) are attached to the target genomic DNA (or fragments) in an amplification reaction. In this process the primer for genomic DNA (or fragments) synthesis (primer specific for a sequence within the target genomic DNA (or fragments)) binds to the target genomic DNA (or fragments) and serves as a starting point for DNA synthesis. The strands are separated and the first plurality of oligonucleotides (comprising the first barcode sequence (BC1mx)) binds to the target genomic DNA (or fragments) (via target specific binding site (TSB)) and serves as a primer and therefore as a starting point for a second strand synthesis. This then leads to incorporation of the first plurality of oligonucleotides ((comprising the first barcode sequence (BC1mx)) into the target genomic DNA (or fragments, thereof). As a result the target genomic DNA (or fragments) comprises said first barcode sequence. In addition to that, the target genomic DNA (or fragments) comprises PS3 and PS4 at the 3' and 5' end of the target nucleic acid respectively. Preferentially the target nucleic acid has the structure (5'-3'): primer binding sequence (PS4) - barcode 1(BC1) - target nucleic acid (sequence) - primer binding sequence (PS3).

In another embodiment of this variant of the invention the target nucleic acid is target genomic DNA (e.g. from single cells) and the first plurality of oligonucleotides comprising a barcode sequence (BC) and a target specific binding site (TSB). Said a target specific binding site (TSB) sequence may be a specific sequence complementary to a sequence comprised in the target nucleic acid. Preferentially, the first plurality of oligonucleotides may have or may comprise the structure (5'-3'): a target specific binding site (TSB) - first barcode sequence (BC1mx) - primer binding sequence (PS3).

Moreover the following components may be additionally provided
- Primer comprise a specific sequence, which is complementary to a sequence within the target genomic DNA (or fragment) and an additional primer binding site (PS4). PS4 is at the 5' end of the oligonucleotide.
- Primer pairs (forward PS1 and reverse primer PS2) specific for the amplification of the second plurality oligonucleotides in step e). The forward primer (PS1) may be specific for primer binding sequence 2 and the reverse primer may be specific for the primer binding sequence (PS2) or vice versa.
- Primer pairs (forward PS4 and reverse PS3) specific for the amplification of the barcoded target nucleic acids in step e). The forward primer may be specific for the primer binding sequence (PS4) and the reverse primer (PS3) is specific for the primer binding sequence (PS3) or vice versa.

There follows step d) attaching to each target genomic DNA (or target genomic DNA fragments) one first oligonucleotide, thereby generating barcoded target genomic DNA (or target genomic DNA fragments) (target nucleic acids), wherein each genomic DNA (or target genomic DNA fragments) comprises a first barcode sequence (BC1mx), wherein this first barcode sequence is different for each target genomic DNA (or target genomic DNA fragments). In this embodiment, the first plurality of oligonucleotides are attached to the target genomic DNA (or target genomic DNA fragments) in an amplification reaction. In this process the first plurality of oligonucleotides serve as primer for genomic DNA (or target genomic DNA fragments) synthesis, bind to the target genomic DNA (or target genomic DNA fragments) and serves as a starting point for nucleic acid synthesis. As a result the first barcode sequence BC1mx is incorporated into the target nucleic acid. After nucleic acid synthesis, the strands are separated and the TSB sequence binds to the target genomic DNA (or target genomic DNA fragments) and serve as a starting point for a second strand synthesis. This then leads to incorporation of the primer binding sequence PS4. As a result the target genomic DNA (or target genomic DNA fragments) comprises said first barcode sequence. In addition to that, the target genomic DNA (or target genomic DNA fragments) comprises the primer binding site PS4 and PS3 at the 3' and 5' end of the target nucleic acid respectively. Preferentially the target genomic DNA (or target genomic DNA fragments) has the structure (5'-3'): primer binding sequence (PS4) - target nucleic acid (sequence) - barcode sequence (BC1mx) - primer binding sequence (PS3).

Independent of whether the target nucleic acids are cDNA, mRNA or genomic DNA, all target nucleic acids generated in Step d) do not comprise overlapping regions with the second plurality of oligonucleotides.

In step e) the second plurality of oligonucleotides and the barcoded target nucleic acids are amplified, thereby generating multiple copies of the second plurality of oligonucleotides and of the target nucleic acids. More specifically, the second plurality of barcodes are amplified using primer specific for the first primer binding sequence(PS1) and the primer binding sequence(PS2). Moreover the barcoded target nucleic acids may be amplified using primer specific for primer binding sequence (PS3) and specific for the primer binding sequence (PS4). The amplification of both (oligonucleotides and barcoded target nucleic acids) may be done by PCR. The outcome of this process are multiple copies of the barcoded target cDNA and multiple copies of each oligonucleotide comprised in the second plurality of oligonucleotides.

There follows step f), which is attaching the second plurality of oligonucleotides to the target cDNA, thereby generating combinatorial barcoded target nucleic acids, wherein each barcoded target nucleic acids comprises a specific combination of the first and second specific barcode sequences, wherein the specific combination of the first and second barcodes serves as partition specific barcode. More specifically the second plurality of oligonucleotides may be attached to the barcoded target nucleic acids by ligation, thereby generating a double stranded target barcoded target nucleic acids. This target nucleic acid comprises two barcodes which can serve as partition specific barcode.

Optionally, the method may additionally comprise the steps breaking the partitions, thereby obtaining a mixture of combinational barcoded target nucleic acids from the partitions.

Sequencing combinatorial barcoded target nucleic acids, thereby obtaining sequencing data comprising sequences of the combination of the first and second barcodes and the sequence of the target nucleic acid. Using the combinational barcode for assigning the target nucleic acid to each partition.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

The current invention discloses a "a method for barcoding of nucleic acids". It is understood that this method is an in vitro method. For this reason the formulation "a method for barcoding of nucleic acids" and the formulation "an in vitro method for barcoding of nucleic acids" can be used interchangeably.

The words "binding" "hybridize" "hybridization" and its grammatical exuviates may be used interchangeably. Hybridization of two nucleic acid strands occurs if they are complementary to each other. Hybridization may occur under conditions known in the art.

As used herein, the term "complementary" refers to the capacity for precise pairing between two nucleotides via Watson and crick base pairing. In explanation, if a nucleotide at a given position of a nucleic acid strand is capable of forming hydrogen bonds with a nucleotide of another nucleic acid strand, then the two nucleic acids are considered to be complementary to one another at that position. Complementarity between two single-stranded nucleic acid molecules may be "partial," in which only some of the nucleotides bind, or it may be complete when total complementarity exists between the single- stranded molecules.

A "primer" as used herein is a single stranded oligonucleotide made of nucleotides, which is able to bind to complementary nucleic acid sequences, referred to as "primer binding site". As disclosed herein, the first and second oligonucleotides may comprise such primer binding sites. It is understood that all primers described in the current invention may serve as starting point for nucleic acid synthesis such as amplification or extension reaction.

The terms "nucleic acid synthesis" is well known in the art. A nucleic acid synthesis (reaction) as disclosed herein may be an extension or an amplification reaction. In Brief: For nucleic acid synthesis a template nucleic acid is provided (e.g. target nucleic acids), which may be single stranded or double stranded. In case double stranded nucleic acid are used initially a first step is the denaturation into single nucleic acid strands (complement and reverse complement) using techniques known in the art. No denaturation step is needed for single stranded nucleic acids. In the next step primer are provided that bind to complementary regions of the nucleic acid strands. The 3'end of the primer is then elongated (or extended) using a polymerase and a complementary strand is generated by filling with complementary nucleotides. As a result a complementary nucleic acid strand is formed. This would be the result of an extension reaction. For further amplification (amplification reaction) denaturation of the double stranded nucleic acid is needed, before another round of nucleic acid synthesis can be initiated

The term "nucleic acid"; "nucleic acid sequence", "nucleic acid molecule" "oligonucleotides" can be used interchangeably and refers to a biopolymer composed of nucleotide monomers covalently bonded in a chain. An amplified nucleic acid may be named as "amplicon". A nucleic acid may be DNA or RNA.

According to the current method, "target nucleic acids" are barcoded. These target nucleic acids are also referred to as "nx" (n = nucleic acid; x=number of the nucleic acid sequence)

"Oligonucleotides" according to the current invention may comprise a "barcode" sequence. A barcode sequence is a short nucleotide sequence for identification purposes. As disclosed herein, in the method of the invention a first and second plurality of oligonucleotides comprising barcode sequences are provided. As disclosed herein, "BC1mx" defines the first plurality of oligonucleotides comprises or consist of a first barcode sequence; (m = specific partition; x=number of the barcode sequence) and "BC2mx" defines the second plurality of oligonucleotides comprises or consist of a second barcode ; (m = specific partition; x=number of the barcode sequence) sequence. In addition to that the first and/or second plurality of oligonucleotides may additionally comprise a third barcode sequence (BC3). Such a barcode could serve e.g. as sample specific barcode.

As disclosed herein, in step a) of the invented method the first (or second, respectively) plurality of oligonucleotides comprises a first (or second, respectively) barcode sequence, wherein each oligonucleotide of the first (or second, respectively) plurality of oligonucleotides comprises a different barcode sequence. In other words, it is assumed that each first and second oligonucleotide comprises a different barcode sequence. It is understood that this assumption is made within the known error rates of methods to synthesize these oligonucleotides. For example: A popular method in the current art is to generate unique or nearly unique barcodes during oligonucleotide synthesis. During oligonucleotide synthesis, nucleotides are stepwise synthesized (nucleotide by nucleotide in a cyclic fashion). Ideally, one discrete nucleotide is added in each cycle in order to generate a specific sequence. But several methods add multiple (at least two) different nucleosides in a synthesis cycle, which results in a stochastic incorporation of one of the multiple different types of nucleosides in the respective cycle. For example, by having two bases with an "N" base (A, T, G or C), you can theoretically generate 16 different barcodes. Similarly, also two or three bases could be incorporated in one cycle. By using this method, large sets of distinct barcode sequences can be generated. All of these methods have in common, that during the initial synthesis of the oligonucleotides, a barcode can occur multiple times. In order to have a set of oligonucleotides with "different" barcodes, it is important to have a larger pool of oligonucleotides with different barcode sequences than the number of oligonucleotides used or incorporated in an experiment. This ensures that the vast majority of barcodes used or incorporated will be different. Throughout this text, the term "different barcode" or "specific barcode" sequence therefore refers to a situation, in which statistically the majority of barcodes of the oligonucleotides will be different. It refers to a situation, wherein at least 50% /90% or 95% of the utilized barcode sequences are different (within the population of first or second oligonucleotides).

Moreover oligonucleotides according to the current invention may comprise an intermediate sequence, which may be referred to as "IM" or "IM sequence". This sequence may be used to introduce an oligonucleotide by hybridization.

The term "a plurality" of something as used herein means two or more.

The term "template switching" or "template switching reaction" is well known in the art. In brief: First, a primer is hybridized to a RNA molecule. This primer serves as priming site for the synthesis of cDNA by an enzyme with reverse transcriptase activity. Once the enzyme reaches the 5' end of the RNA template, a subset of reverse transcriptase (such as the MMLV reverse transcriptase) is capable of adding one or more additional nucleotides to the 3' end of the newly synthesized cDNA (mostly deoxycytidines). This allows the template switching oligonucleotide (TSO) to bind to these deoxycytidines. Subsequently, the reverse transcriptase can switch the template and continue cDNA synthesis. Examples for protocols using reverse transcription and template switching can be found in in Zhu et al, 2001 and Wellenreuther et al., 2004.

### Examples

The following examples are intended for a more detailed explanation of the invention but without restricting the invention to these examples.

### Example 1:

To prove the feasibility of the method, we evaluated the protocol in separate wells of a 96-well plate. First, cDNA was synthesized using 6000 cells (K562 cell line) in each well using the protocol of Hagemann-Jensen et al, 2020 with modification (cells were not prelysed but the final RT buffer was added directly onto the cells). As template switching oligo, we used an oligonucleotide corresponding to SEQ02 which contains a random sequence of 12 nucleotides (first barcode).

After cDNA synthesis, the following reagents were added to each well: 1 pmol of SEQ01 (containing a random sequence of 16 nucleotides [second barcode]), 1 µM of primers SEQ04 and SEQ07, 0.01 µM of primers SEQ05 and SEQ06, and 25 µl of KAPA HiFi HotStart ReadyMix (Cat No. 07958927001, Roche Molecular Systems, Basel, Switzerland) in a 50 µl reaction. The reaction was subjected to the following cycling conditions: 45 seconds at 98°C; 12 cycles of 20 seconds at 98°C - 30 seconds at 69°C and 1 minute at 72°C; 12 cycles of 20 seconds at 98°C - 30 seconds at 63°C and 1 minute at 72°C; 1 minute at 72°C; hold at 4°C.

After cycling, each sample was subjected to 0.6x cleanup using SPRIselect beads (Cat. No. B23317, Beckman Coulter, Brea, CA, USA). Only combinations in with barcoded cDNA (Sample # 1-6) was combined with the second oligonucleotide lead to significant amplification whereas the controls (Sample # 7 and 8) did not show amplification (Yield column in Fig 6A).

Next, samples and analyzed on an Agilent 4200 TapeStation System using D5000 or High Sensitivity D5000 Screen Tapes (Cat. No. 5067-5588 and 5067-5592, Agilent, Santa Clara, CA, USA). The size of the products of samples 1-6 showed the typical distribution of cDNA, therefore strongly suggesting that barcoded cDNA (target nucleic acid with first barcode) was successfully combined with the second oligonucleotide (barcoded oligonucleotide).

We next analyzed the obtained product by sequences. For this, samples were subjected to library prep using the 10x Genomics 5' Library Construction Kit (PN-220111, 10x Genomics, Pleasanton, CA, USA). Sample index PCR was conducted using the Sample Index PCR Primer (PN-220111, 10x Genomics, Pleasanton, CA, USA) and the Single Index Plate T Set A (PN-2000240, 10x Genomics, Pleasanton, CA, USA). The libraries were then sequenced on an Illumina MiSeq Nano Cartridge (MiSeq Reagent Nano Kit v2, Illumina, San Diego, CA, USA).

The results of the sequencing run is summarized in the bottom table of Fig 6A. The column % reads containing both barcodes refers to the relative abundance of index 2 reads which contain the barcode plus the first four bases. This combination is only possible of the amplified second barcode has successfully been combined with the barcoded target nucleic acid. Such a combination occurred in an unexpectedly high number of cases (almost 95 % of all obtained reads). Additionally, the obtained reads were typical for Gene Expression/ cDNA analysis as indicated by the number of mapped reads, mapped reads to genes, exons and intergenic.

## Claims

1. A method for barcoding of nucleic acids comprising the steps:
a) Providing
i. a plurality of biological particles comprising target nucleic acids,
ii. a first plurality of oligonucleotides comprising a first barcode sequence, wherein each oligonucleotide of the first plurality of oligonucleotides comprises a different barcode sequence and
iii. a second plurality of oligonucleotides comprising second barcode sequence, wherein each oligonucleotide of the second plurality of oligonucleotides comprises a different barcode sequence
b) Partitioning the plurality of biological particles such that each partition comprises a biological particle and a subset of the first and second oligonucleotides
c) Optionally releasing the target nucleic acids of the biological particles into the partition
d) Attaching to each target nucleic acid one first oligonucleotide, thereby generating barcoded target nucleic acids, wherein each target nucleic acid comprises a first barcode sequence, wherein this first barcode sequence is different for each target nucleic acid
e) Amplifying the second plurality of oligonucleotides and the barcoded target nucleic acids, thereby generating multiple copies of the second plurality of oligonucleotides and of the target nucleic acids
f) Attaching the second plurality of oligonucleotides to the target nucleic acids, thereby generating combinatorial barcoded target nucleic acids, wherein each target nucleic acid comprises a specific combination of the first and second specific barcode sequences, wherein the specific combination of the first and second barcodes serves as partition specific barcodes

2. A method according to claim 1, Wherein the method additionally comprises the steps
g) Breaking the partitions, thereby obtaining a mixture of combinational barcoded target nucleic acids from the partitions
h) Sequencing the combinatorial barcoded target nucleic acids, thereby obtaining sequencing data comprising sequences of the combination of the first and second barcodes and the sequence of the target nucleic acid
i) Using the combinational barcode for assigning the target nucleic acid to each partition.

3. A method according to claim any of the claims 1 and 2, wherein the attachment of the second oligonucleotide in step f) is by hybridization, extension, amplification or ligation

4. A method according to any of the claims 1-3, Wherein the first and second plurality of oligonucleotides additionally comprise an intermediate sequence (IM), wherein the IM sequences are complementary to each other, wherein the IM sequence is incorporated in the barcoded target nucleic acid and herein the second plurality of oligonucleotides are attached in step e) by complementary hybridization of the IM sequences, followed by an extension reaction wherein the second plurality of oligonucleotides serve as primer

5. A method according to any of the claims 1-4, wherein said biological particles provided in step a) are selected from the group consisting of: single cells bacteria, viral particles

6. A method according to any of the claims 1-5, wherein plurality of biological particles provided in step a) is a population of single cells and wherein in step c) the single cells are lysed and the target nucleic acid are released in the partition.

7. A method according to claim any of the claims 1-6, wherein the target nucleic acids are genomic DNA or mRNA

8. A method according to claim 7, wherein the target nucleic acids are mRNA and
wherein the first plurality of oligonucleotides additionally comprise a template switching sequence and wherein the first oligonucleotides are attached in step d) by a template switching reaction

9. A method according to claim 7, wherein the target nucleic acids are genomic DNA
wherein the genomic DNA is fragmented after step c), wherein the first oligonucleotides are attached in step d) by ligation
